(19) Europäisches Patentamt
European Patent Office
Office européen des brevets

(11) EP 2 216 406 B1

(12) **EUROPEAN PATENT SPECIFICATION**

(45) Date of publication and mention of the grant of the patent:
**15.01.2014 Bulletin 2014/03**

(21) Application number: **08855276.5**

(22) Date of filing: **27.11.2008**

(51) Int Cl.:
***C12N 15/09*** *(2006.01)*
***C12N 15/10*** *(2006.01)*
***C12N 15/11*** *(2006.01)*
***C12M 1/00*** *(2006.01)*
***G01N 33/53*** *(2006.01)*
***C12N 15/113*** *(2010.01)*

(86) International application number:
**PCT/JP2008/071576**

(87) International publication number:
**WO 2009/069711 (04.06.2009 Gazette 2009/23)**

(54) **CARRIER, METHOD AND REAGENT FOR OBTAINING SMALL RNA**

SUBSTRAT, VERFAHREN UND REAGENZ ZUR GEWINNUNG VON sRNA

SUPPORT, PROCÉDÉ ET RÉACTIF DESTINÉS À OBTENIR UN PETIT ARN

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.11.2007 JP 2007311317**
**16.05.2008 JP 2008129068**

(43) Date of publication of application:
**11.08.2010 Bulletin 2010/32**

(73) Proprietors:
- **Wako Pure Chemical Industries, Ltd.**
  **Osaka-shi**
  **Osaka 540-8605 (JP)**
- **Sumitomo Bakelite Co., Ltd.**
  **Shinagawa-ku**
  **Tokyo 140-0002 (JP)**

(72) Inventors:
- **NISHIBU, Takahiro**
  **Amagasaki-shi**
  **Hyogo 661-0963 (JP)**
- **FUNAOKA, Sohei**
  **Tokyo 140-0002 (JP)**
- **HAMAGUCHI, Yuzo**
  **Tokyo 140-0002 (JP)**

(74) Representative: **von Kreisler Selting Werner**
**Deichmannhaus am Dom**
**Bahnhofsvorplatz 1**
**50667 Köln (DE)**

(56) References cited:
**WO-A1-2005/029095 WO-A1-2007/037069**
**WO-A1-2007/037069 JP-A- 2006 184 015**

- **PARK J ET AL: "EVALUATION OF 2-METHACRYLOYLOXYETHYL PHOSPHORYLCHOLINE POLYMERIC NANOPARTICLE FOR IMMUNOASSAY OF C-REACTIVE PROTEIN DETECTION", ANALYTICAL CHEMISTRY, AMERICAN CHEMICAL SOCIETY, US, vol. 76, no. 9, 1 May 2004 (2004-05-01), pages 2649-2655, XP001196758, ISSN: 0003-2700, DOI: 10.1021/AC035321I**
- **IKEDA K ET AL: "Detection of the argonaute protein Ago2 and microRNAs in the RNA induced silencing complex (RISC) using a monoclonal antibody", JOURNAL OF IMMUNOLOGICAL METHODS, ELSEVIER SCIENCE PUBLISHERS B.V.,AMSTERDAM, NL, vol. 317, no. 1-2, 20 December 2006 (2006-12-20), pages 38-44, XP025158240, ISSN: 0022-1759, DOI: 10.1016/J.JIM.2006.09.010 [retrieved on 2006-12-20]**
- **MEISTER ET AL: "Identification of Novel Argonaute-Associated Proteins", CURRENT BIOLOGY, CURRENT SCIENCE, GB, vol. 15, no. 23, 6 December 2005 (2005-12-06), pages 2149-2155, XP005210467, ISSN: 0960-9822, DOI: 10.1016/J.CUB.2005.10.048**
- **IKEDA KEIGO ET AL.: 'Detection of the argonaute protein Ago2 and microRNAs in the RNA induced silencing complex (RISC) using a monoclonal antibody.' JOURNAL OF IMMUNOLOGICAL METHODS vol. 317, 2006, pages 38 - 44, XP025158240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MIKIKO SHIOMI: 'Hito Argonaute2 (hAgo2) ni Taisuru Monoclonal Kotai no Sakusei' WAKO JUN'YAKU JIHO vol. 74, no. 4, 2006, pages 2 - 4**

## Description

### Technical field

The present invention relates to a carrier for obtaining a small RNA and a method for obtaining a small RNA and/or a nucleic acid which is targeted by the small RNA using the same.

### Background Art

Micro RNA (miRNA) is a group of functional low molecular weight RNA comprising about 22 nucleotides, and has attracted attention as a guide molecule toward controlling post-transcriptional gene expression. Especially, the miRNA has been known to be involved deeply in cell differentiation and malignant alteration, and the like. Furthermore, after having matured by passing several steps in a cell, the miRNA forms a complex called as RISC (RNA-induced silencing complex) with protein. And, it has been reported that the miRNA incorporated into the RISCfinds out a target mRNA depending on the nucleotide sequence thereof and gives rise to cleavage or translational repression of the mRNA. On the other hand, it has been also known that an Ago2 protein which is a major component of the RISC is capable of binding with miRNA, and anti-Ago2 antibody is also in the market as an antibody which is capable of recognizing Ago2 protein. And a method for obtaining miRNA by immunoprecipitation method using said anti-Ago2 antibody has been reported by Ikeda et al. in Journal of Immunological Methods, 10417, 2006, etc. However, in the anti-Ago2 antibody-immobilized carrier employed here, the anti-Ago2 antibody was immobilized on the carrier with the use of affinity for protein A, and therefore, the method had such problems that the anti-Ago2 antibody might be eluted depending on the reaction condition, and that the method required the complicated operations such that, in addition to preparation of the protein A-immobilized carrier, the anti-Ago2 antibody has to be prepared and to make them react with RISC. Therefore, a stable anti-Ago2 antibody-immobilized carrier which does not require such a complicated operation and yet does not elute anti-Ago2 antibody has been desired. In addition, as to the immunoprecipitation method using said anti-Ago2 antibody-immobilized carrier, since development of the method has just started, development of a method for obtaining small RNA such as miRNA in high yield yet in high purity using said method has also been desired.

[Non-patent reference 1]: Ikeda et al., Journal of Immunological Methods, 10417, 2006

International application WO 2007/037069 A1 discloses a carrier suitable for obtaining a biologically active substance and comprising a core particle bearing a polymerizable functional group on its surface (to allow a covalent bond between a polymer compound and the surface of the core particle), a layer comprising a polymer compound, and a physiologically active substance having affinity to a biologically active substance immobilized, wherein the polymer layer is formed on the surface of said particle by applying a solution of the polymer compound to the surface of the core particle.

Japanese application JP 2006/184015 A discloses a carrier suitable for obtaining a biologically active substance and comprising a core particle/solid substrate having a polymer layer formed on its surface, said polymer having a phosphocholine and an active ester group and wherein the polymer layer is formed on the surface of said particle by immersing the substrate into a solution of the polymer.

Park et al. (Park et al., Analytical Chemistry 2004, 76(9): 2649-2655) discloses nano-particles suitable for obtaining C-Reactive protein and comprising a core particle bearing a polymerizable functional group or a chain transfer group on its surface, a layer comprising a polymer compound, and a physiologically active substance having affinity to CRP immobilized on the surface of the particle and wherein the polymer layer is formed on the surface of said particle by mixing poly(L-lactic acid) with a copolymer.

### Disclosure of the Invention

### Problem to be Solved by the Invention

An object of the present invention is to provide a carrier which enables to obtain a small RNA in high yield yet in high purity, a simple method for obtaining a small RNA and/or a nucleic acid which is targeted by the small RNA (hereinafter, optionally abbreviated as target nucleic acid of small RNA) using the same, and a reagent and a kit comprising the above-described carrier.

### Means for Solving Problem

The present invention has been made based on the results of intensive studies carried out by the present inventors in view of the above-described situation, and relates to (1) a carrier for obtaining a small RNA produced by introducing a polymerizable functional group or a chain transfer group to the surface of a core particle, mixing said

particle with a polymerizable monomer, then promoting polymerization reaction to form a layer comprising a polymer compound on the surface of said particle, and immobilizing a physiologically active substance having affinity to a small RNA-binding protein on the surface of the particle; (2) the use of the above-described carrier in a method for obtaining a small RNA; (3) a method for obtaining a small RNA by using the above-described carrier, wherein the method comprises the following steps:

(i) introducing a polymerizable functional group or a chain transfer group to the surface of core particle, mixing said particle with a polymerizable monomer, subsequently promoting polymerization reaction to form a layer comprising a polymer compound on the surface of said particle, and immobilizing a physiologically active substance having affinity to small RNA-binding protein on the surface of said particle to form a carrier, and contacting the carrier with a complex of said small RNA-binding protein and a small RNA (small RNA-binding protein-small RNA complex) to form a bound substance of said physiologically active substance and said small RNA-binding protein-small RNA complex on the surface of said carrier,
(ii) isolating the obtained carrier having a bound substance of physiologically active substance and small RNA-binding protein-small RNA complex on the surface thereof, and
(iii) eluting the small RNA from said bound substance of physiologically active substance and small RNA-binding protein-small RNA complex; (4) a reagent for obtaining a small RNA and/or a target nucleic acid of small RNA, comprising the above-described carrier; (5) a reagent kit for obtaining a small RNA and/or a target nucleic acid of small RNA, comprising the above-described reagent; and (6) the use of the above-described carrier in a method for obtaining a target nucleic acid of small RNA; and (7) a method for obtaining a target nucleic acid of small RNA by using the above-described carrier, wherein the method comprises the following steps:

(i) introducing a polymerizable functional group or a chain transfer group to the surface of core particle, mixing said particle with a polymerizable monomer, subsequently promoting polymerization reaction to form a layer comprising a polymer compound on the surface of said particle, and immobilizing a physiologically active substance having affinity to small RNA-binding protein on the surface of said particle to form a carrier, and contacting the carrier with a complex of said small RNA-binding protein,small RNA and a target nucleic acid of small RNA (small RNA-binding protein-small RNA-target nucleic acid of small RNA complex) to form a bound substance of said physiologically active substance and said small RNA-binding protein-small RNA-target nucleic acid of small RNA complex on the surface of said carrier,
(ii) isolating the obtained carrier having the bound substance of physiologically active substance and small RNA-binding protein-small RNA -target nucleic acid of small RNA complex on the surface thereof, and
(iii) eluting the bound substance of small RNA-target nucleic acid of small RNA from said bound substance of physiologically active substance and small RNA-binding protein-small RNA - target nucleic acid of small RNA complex.

**Effect of the Invention**

As for the carrier of the present invention, for example, contaminating proteins in a cell lysate are hardly adsorbed on the surface thereof, and therefore inhibition of binding reaction between the physiologically active substance on the surface of the carrier and the small RNA-binding protein (for example, inhibition of antigen-antibody reaction between Ago2 and anti-Ago2 antibody) hardly occurs. In addition, since nonspecific adsorption of protein rarely occurs, it is easy to determine the recovery of small RNA-binding protein (for example, Ago2) from the carrier surface. Further, according to the method for obtaining a small RNA of the present invention using the carrier of the present invention, small RNA can be obtained in high yield yet in high purity, because the disincentive caused by nonspecifically adsorbed protein does not become significant when RNA is purified. Furthermore, according to the method for obtaining a small RNA of the present invention, as compared with conventional methods, the step in which the physiologically active substance such as antibody is immobilized to the carrier can be skipped, and addition of a blocking agent and the like for preventing nonspecific adsorption is also not necessary, and small RNA can be obtained by simple operation. Furthermore, by using the carrier of the present invention, a target nucleic acid of small RNA can be obtained easily yet efficiently by the same procedures as in the method for obtaining a small RNA.

**Brief Description of Drawings**

[Fig. 1] Fig. 1 is the results of gel electrophoresis of the solutions obtained, using HeLa cell as a sample, by conducting nonspecific adsorption experiment by immunoprecipitation method using mouse IgG antibody-immobilized carrier

which was prepared using various types of carriers.

[Fig. 2] Fig. 2 is the results of gel electrophoresis of the solutions obtained, using HeLa cell as a sample, by conducting Ago2 adsorption experiment by immunoprecipitation method using anti-Ago2 antibody-immobilized carrier which was prepared using various types of carriers.

[Fig. 3] Fig. 3 is the results of gel electrophoresis of the solution (the solution containing miRNA) obtained, using HeLa cell as a sample, by immunoprecipitation method employing various concentrations of the anti-human Ago2 antibody-immobilized carrier.

[Fig. 4] Fig. 4 is the results of gel electrophoresis of the solutions which were obtained, using the Total RNA solution of HeLa cell, the solution obtained by immunoprecipitation method employing mouse IgG-immobilized carrier, and the solution obtained by immunoprecipitation method employing anti-human Ago2 antibody-immobilized carrier, the RNA included in each solution was subjected to reverse transcription reaction, and further subjected to the PCR reaction.

[Fig. 5] Fig. 5 is the results of the measurement by quantitative PCR method for the amounts of MiR-122 and miR-21 in the Ago2 immunoprecipitated RNA obtained from HepG2 cell in which miR-122 double-stranded RNA or firefly luciferase siRNA (GL3) had been introduced.

[Fig. 6] Fig.6 is the results of the measurement by quantitative PCR method for the amounts of AldoA, CAT-1, and GAPDH mRNA in the Ago2 immunoprecipitated RNA and total RNA obtained from HepG2 cell in which miR-122 double-stranded RNA or firefly luciferase siRNA (GL3) had been introduced.

## DESCRIPTION OF REFERENCE NUMERALS

Lane 1 in Fig. 1 represents an electrophoresis result of a molecular weight marker, Lane 2 represents the electrophoresis result when a carrier produced by the particle involved in the present invention is employed, Lane 3 represents the electrophoresis result when the carrier produced by Pierce is employed, Lane 4 represents the electrophoresis result when the carrier produced by GE is employed, and Lane 5 represents the electrophoresis result when the carrier produced by DYNAL is employed, respectively. In addition, upper arrow represents a mouse IgG heavy chain, and lower arrow represents mouse IgG light chain, respectively.

Lane 1 in Fig. 2 represents the electrophoresis result of a molecular weight marker, Lane 2 represents the electrophoresis result when an anti-Ago2 antibody-immobilized carrier employing the particle involved in the present invention is employed, Lane 3 represents the electrophoresis result when the anti-Ago2 antibody-immobilized carrier produced by Pierce is employed, Lane 4 represents the electrophoresis result when the anti-Ago2 antibody-immobilized carrier produced by GE is employed, and Lane 5 represents the electrophoresis result when the anti-Ago2 antibody-immobilized carrier produced by DYNAL is employed. In addition, the arrow represents Ago2, an Ago2 anti-weight chain, and Ago2 antibody light chain sequentially from a top.

Lane 1 in Fig. 3 represents the electrophoresis result of a molecular weight marker, Lane 2 represents the electrophoresis result when a synthetic RNA Oligo (22nt) 0.25ng is employed, Lane 3 represents the electrophoresis result when a synthetic RNA Oligo (22nt) 0.5ng is employed, Lane 4 represents the electrophoresis result when a synthetic RNA Oligo (22nt) 1ng is employed, Lane 5 represents the electrophoresis result when a synthetic RNA Oligo (22nt) 2ng is employed, Lane 6 represents the electrophoresis result when the RNA obtained by anti-human Ago2 antibody 5 μg/2mg carrier is employed, Lane 7 represents the electrophoresis result when the RNA obtained by anti-human Ago2 antibody 10 μg/2mg carrier is employed, and Lane 8 represents the electrophoresis result when the RNA obtained by anti-human Ago2 antibody 20 μg/2mg carrier is employed, respectively.

Lane 1 in Fig. 4 represents the electrophoresis result derived from template cDNA = Total RENAL μg, and Lane 2 represents the electrophoresis result derived from immunoprecipitation RNA (1 x 107 cell) employing template cDNA = mouse IgG-immobilized carrier, and Lane 3 represents the electrophoresis result derived from immunoprecipitation RNA (1 x 107 cell) employing template DNA anti-human Ago2-immobilized carrier, respectively.

### Best Mode for Carrying Out the Invention

The particle involved in the present invention is a particle which is produced by introducing a polymerizable functional group or a chain transfer group onto the surface of a core particle, mixing the particle with a polymerizable monomer, and promoting the polymerization reaction to form a layer comprising a polymer compound on the surface of a core particle. The above-described polymerizable monomer is the one which comprises an ethylenically unsaturated polymerizable monomer (a) having a functional group which can immobilize a physiologically active substance, and if necessary, the one which further comprises an ethylenically unsaturated polymerizable monomer (b) is preferable. The polymer compound formed on the surface of a core particle has a property of immobilizing a specific physiologically active substance. In addition, the ethylenically unsaturated polymerizable monomer having an alkylene oxy group (b) has a property of inhibiting nonspecific adsorption of protein and the like. Furthermore, since the polymer compound is

formed on the surface of the particle utilizing a polymerizable functional group or a chain transfer group which has formed a covalent bond to the surface of core particle, it is possible to graft said polymer compound densely on the surface of the core particle. In the grafted particle thus obtained, elution of said polymer compound from the particle during washing step will never take place.

The functional group of the ethylenically unsaturated polymerizable monomer (a) having a functional group involved in the present invention which immobilizes physiologically active substance includes, but not limited thereto, a chemically active group, a receptor group, a ligand group, and the like. Specific example includes an aldehyde group, an active ester group, an epoxy group, a vinyl sulfone group, a group derived from biotin, a thiol group, an amino group, an isocyanate group, an isothiocyanate group, a hydroxyl group, an acrylate group, a maleimide group, a hydrazide group, an azido group, an amide group, a sulfonate group, a group derived from streptavidin, a metal chelate, etc. Among them, from the view point of reactivity with amino group which is contained in many physiologically active substances, an aldehyde group, an active ester group, an epoxy group and a vinyl sulfone group are preferable; and from the view point of high coupling constant with physiologically active substance, a group derived from biotin is preferable. Among others, the active ester group is the most preferable from the view point of storage stability.

The ethylenically unsaturated polymerizable monomer (a) having a functional group which can immobilize physiologically active substance involved in the present invention is not particularly limited, but preferably a compound represented by the following general formula [1], wherein a (meth)acrylic group and an active ester group are bonded through a chain of alkylene oxy group or alkylene group having 1 to 10 carbon atoms. Especially, the chain of the alkylene oxy group has a nature in itself of inhibiting nonspecific adsorption of protein. For this reason, the monomer in which the (meth)acrylic group and the active ester group are bound through the chain of the alkylene oxy group has a nature of immobilizing a physiologically active substance, together with a nature of inhibiting nonspecific adsorption of protein. Therefore, the polymer of such a monomer can be suitably employed as a polymer which forms a layer on the surface of the particle, even if it is a homopolymer, so long as it has a reactive functional group on the terminal of at least one side.

[Chemical Formula 1]

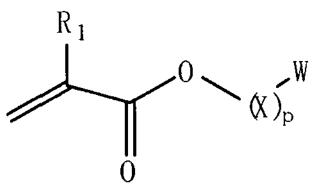

[1]

(In the above formula, $R_1$ represents a hydrogen atom or a methyl group. X represents an alkylene oxy group or an alkylene group having 1 to 10 carbon atoms. W represents an active ester group. p represents an integer of 1 to 100. When p is 2 to 100, each repeating unit X may be either identical or different.)

In formula [1], the number of carbon atoms of alkylene oxy group and alkylene group X is 1 to 10, preferably 1 to 6, more preferably 2 to 4, still more preferably 2 to 3, and most preferably 2. The repetition number p of alkylene oxy group or alkylene group X is an integer of 1 to 100, more preferably an integer of 2 to 90, and most preferably an integer of 2 to 80. In addition, when p is 2 to 100, the number of carbon atoms of p pieces of alkylene oxy group or alkylene group to be repeated may be either identical or different.

The above-described alkylene group may be any of straight, branched, and cyclic, and specifically includes, for example, methylene group, ethylene group, propylene group, trimethylene group, butylene group, 1-ethylethylene group, 2-methyltrimethylene group, and 2-ethyltrimethylene group, hexylene group, cyclopropylene group, cyclobutylene group, cyclopentylene group, cyclohexylene group and so on, and among them, ethylene group is preferable. The alkylene oxy group includes the ones in which an oxy group is bound to the above-described alkylene group, and among them, ethylene oxy group is preferable. As to alkylene oxy group and alkylene group X in the formula [1], among those described above, alkylene oxy group is preferable, and ethylene oxy group is particularly preferable.

The "active ester group" to be used in the present invention is ester groups which have been activated to the nucleophilic reaction by possessing a highly acidic electron-attracting group in one substituent of the ester group, i.e., those which are commonly used in the fields of various types of chemical syntheses, for example, polymer chemistry, peptide synthesis, and the like, as those meaning an ester group having a high reactivity. Practically, the group includes, for example, an ester group formed by dehydrating condensation between a carboxyl group in a certain compound and a compound which has a hydroxyl group or a mercapto group. As to the active ester group which is formed by introducing an ester group, phenol esters, thiophenol esters, N-hydroxyamine esters, esters of heterocyclic hydroxy compound and the like are known to have active ester group having far higher activity compared with alkyl ester and the like.

Such active ester group includes, for example, p-nitrophenyl active ester group, N-hydroxysuccinimide active

ester group, phthalic imide active ester group, 5-norbornene-2, 3-dicarboximide active ester group and the like; among them, p-nitrophenyl active ester group or N-hydroxysuccinimide active ester group is preferable, and p-nitrophenyl active ester group is particularly preferable.

The composition ratio of the monomer unit originating in the ethylenically unsaturated polymerizable monomer (a) having a functional group capable of immobilizing physiologically active substance involved in the present invention in the polymer compound is not particularly limited, but is preferably 1 to 99.7 mol%, more preferably 1 to 80 mol%, and particularly preferably 1 to 70 mol%.

The ethylenically unsaturated polymerizable monomer (b) having an alkylene oxy group to be used in the present invention is not particularly limited in the structure thereof, but preferably a compound represented by the general formula [2], which is composed of a (meth)acryl group and a chain of alkylene oxy group Y having 1 to 10 carbon atoms.

[Chemical Formula 2]

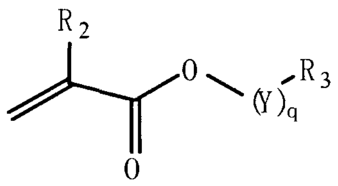

[2]

(In the above formula, $R_2$ represents a hydrogen atom or a methyl group, and $R_3$ represents a hydrogen atom or an alkyl group having 1 to 20 carbon atoms. Y represents an alkylene oxy group having 1 to 10 carbon atoms, and q represents an integer of 1 to 100. Each repeating unit Y may be either identical or different.)

In formula [2], the number of carbon atoms of alkylene oxy group Y is 1 to 10, preferably 1 to 6, more preferably 2 to 4, further more preferably 2 to 3, and most preferably 2. The repetition number of alkylene oxy group q is not particularly limited, but preferably an integer of 1 to 100, more preferably an integer of 2 to 100, further more preferably an integer of 2 to 95, and most preferably an integer of 20 to 90. When the repetition number is 2 to 100, the number of carbon atoms of repeated q pieces of alkylene oxy groups Y may be either identical or different.

The ethylenically unsaturated polymerizable monomer (b) having an alkylene oxy group includes, for example, methoxy polyethylene glycol (meth)acrylate, ethoxy polyethylene glycol (meth)acrylate, 2-hydroxyethyl (meth)acrylate, 2-hydroxypropyl (meth)acrylate and monosubstituted ester of the hydroxyl group thereof, 2-hydroxybutyl (meth)acrylate and monosubstituted ester of the hydroxyl group thereof, glycerol mono-(meth)acrylate, (meth)acrylate having polypropylene glycol as a side chain, 2-methoxyethyl (meth)acrylate, 2-ethoxyethyl (meth)acrylate, methoxy diethylene glycol (meth)acrylate, ethoxy diethyleneglycol (meth) acrylate, ethoxy polyethylene glycol (meth)acrylate, and the like; however, from the viewpoints of less nonspecific adsorption of components other than the desired physiologically active substance and availability thereof, methoxy polyethylene glycol methacrylate or ethoxy polyethylene glycol methacrylate is preferable, and methoxy polyethylene glycol methacrylate is more preferable.

The composition ratio of the monomer unit originating in the ethylenically unsaturated polymerizable monomer (b) having an alkylene oxy group involved in the present invention in the polymer compound is not particularly limited, but preferably 0.3 to 99 mol%, more preferably 20 to 99 mol%, and most preferably 30 to 99 mol%.

The polymerizable functional group to be introduced into the surface of the particle involved in the present invention includes vinyl group, allyl group, methacryl group, epoxy group, styrene group, and the like; and above all, methacryl group is preferable from the viewpoint of superior polymerizability.

The chain transfer group to be introduced into the surface of the particle involved in the present invention includes mercapto group, amino group, and the like; and above all, mercapto group is preferable from the viewpoint of superior reactivity.

Method for introducing a polymerizable functional group or a chain transfer group into the surface of the particle is not particularly limited, but preferably such a method in which a covalent bond is formed between silane coupling agent having a polymerizable functional group or a chain transfer group and functional group on the surface of the core particle.

The silane coupling agent having a polymerizable functional group includes, for example, (3-methacryloxypropyl)dimethylmethoxysilane, (3-methacryloxypropyl)diethylmethoxysilane, (3-methacryloxypropyl)dimethylethoxysilane, (3-methacryloxypropyl)diethylethoxysilane, (3-methacryloxypropyl)methyldimethoxysilane, (3-methacryloxypropyl) ethyldimethoxysilane, (3-methacryloxypropyl)methyldiethoxysilane, (3-methacryloxypropyl)ethyldiethoxysilane, (3-methacryloxypropyl)trimethoxysilane, (3-methacryloxypropyl)triethoxysilane, and so on; and above all, (3-methacryloxypropyl)trimethoxysilane and (3-methacryloxypropyl)triethoxysilane are preferable from the viewpoints of reactivity and availability thereof. These silane coupling agents may be used alone or in combination of two or more types.

The silane coupling agent having a chain transfer group includes, for example, (3-mercaptopropyl)trimethoxysilane, (3-mercaptopropyl)methyldimethoxysilane, (3-mercaptopropyl)dimethylmethoxysilane, (3-mercaptopropyl)triethoxysilane, (3-mercaptopropyl)methyldiethoxysilane, (3-mercaptopropyl)dimethylethoxysilane, (mercaptomethyl)trimethoxysilane, (mercaptomethyl)methyldimethoxysilane, (mercaptomethyl)dimethylmethoxysilane, (mercaptomethyl) triethoxysilane, (mercaptomethyl)methyldiethoxysilane, (mercaptomethyl)dimethylethoxysilane, and so on; and above all, (3-mercaptopropyl)trimethoxysilane and (3-mercaptopropyl)triethoxysilane are preferable from the viewpoint of availability. These mercaptosilane compounds may be used alone or in combination of two or more types.

Method for forming a covalent bond between a polymerizable functional group or a chain transfer group and a functional group on the surface of a core particle using a silane coupling agent having a polymerizable functional group or a chain transfer group is not particularly limited, but it is carried out, for example, by adding a silane coupling agent having a polymerizable functional group or a chain transfer group is added to an acidic aqueous solution of pH 2 to 4 so as to be 0.01 to 1.0 mol/L, hydrolyzing by mixing under stirring, charging a core particle and stirring at 10 to 100°C for 5 to 180 minutes, then drying the particle by heating at 20 to 100°C. The ratio of the core particle and the silane coupling agent having a polymerizable functional group or a chain transfer group to be used is not particularly limited, but usually 0.1 to 10 mmol of the silane coupling agent having a polymerizable functional group or a chain transfer group per 1 g of the core particle is used. The acidic aqueous solution is not particularly limited, but aqueous acetic acid, aqueous hydrochloric acid and the like are employed. Above all, aqueous acetic acid which is relatively easy in handling is preferable.

Method for introducing a polymerizable functional group or a chain transfer group to the surface of a core particle, mixing said particle with a polymerizable monomer, then promoting a polymerization reaction is not particularly limited, but it may be carried out, for example, by adding the core particle into a solvent in which a polymerizable monomer and polymerization initiator have been dissolved, and heating under stirring at 0 to 80°C for 1 to 30 hours. Subsequently, the particles having the surface which has been coated with a polymer compound are filtered under reduced pressure, and washed then dried.

Ratio of the core particle, the polymerizable monomer and the polymerization initiator to be used is not particularly limited, and usually the 0.1 to 10 mmol of the polymerizable monomer and 0.01 to 10 mmol of the polymerization initiator for 1g of the core particle is employed.

The solvent may be the one which can dissolve respective polymerizable monomers, and includes, for example, alcohols such as methanol, ethanol, isopropanol, n-butanol, t-butyl alcohol and n-pentanol, benzene, toluene, tetrahydrofuran, dioxane, dichloromethane, chloroform, cyclohexanone, N,N-dimethylformamide, dimethylsulfoxide, methyl acetate, ethyl acetate, butyl acetate, methyl ethyl ketone, methyl butyl ketone, ethylene glycol monoethyl ether, ethylene glycol monomethyl ether, ethylene glycol monobutyl ether, etc. These solvents are used alone or in combination of two or more types.

The polymerization initiator is not particularly limited, and includes, for example, azo compounds such as 2,2'-azobisisobutyl nitrile (hereinafter referred to as "AIBN"), 1,1'-azobis(cyclohexane-1-carbonitril), organic peroxides such as benzoyl peroxide and lauryl peroxide.

As to the chemical structure of the polymer compound to be used for the surface of the particle involved in the present invention, linkage type thereof may be any form of random, block, grafted, or the like, so long as it is a (co) polymer containing monomer unit derived from ethylenically unsaturated polymerizable monomer that has at least a functional group which can immobilizes a physiologically active substance.

Material of the core particle to be used in the present invention is not particularly limited, and any material can be used regardless of organic substance or inorganic substance, however the one which has (or can be introduced with) a group capable of reacting with a silane coupling agent is preferable. As to an organic carrier, in addition to the porous agarose particles (trade name: Sepharose) and dextran particles (trade name: Sephadex) which are employed as a carrier of affinity chromatography, the particle which consists of polyacrylamide gel (trade name: Bio-Gel P, Bio-Rad Laboratories, Inc.), polystyrene, and ethylene-maleic anhydride copolymer, polymethylmethacrylate, etc. can be employed. On the other hand, as to an inorganic substance, inorganic oxides are preferable due to high strength of the particle itself. Among them, silicon dioxide is most preferable due to easy handling. Further, the particle size is not limited at all, and can be selected appropriately depending on the purpose and the use. This means that a particle having any size can be produced only if size of the particle as a core is selected. This point is a great advantage in comparison with the method of producing particles by emulsion polymerization or suspension polymerization, in which controlling the size of the particles is difficult. When the particle is practically used, the particle having a particle size of about several nm to 100 $\mu$m is preferable, although the particle size varies depending on the use.

The particle involved in the present invention is obtained as mentioned above, and the property of inhibiting the nonspecific adsorption of components other than the target protein is enhanced by adding a component which comprises an alkylene oxy group (alkylene glycol residue) as a component of the layer containing a polymer compound on the surface of the particle. Moreover, because a polymer compound is formed on the surface of the particle using a polymerizable functional group or a chain transfer group which has been formed a covalent bond to the surface of the core particle, it is possible to graft said polymer compound on the surface of the core particle in high density. The grafted

particle thus obtained is superior particle, because nonspecific adsorption is extremely low; and elution of said polymer compound from the particle during washing step never takes place.

The small RNA-binding protein involved in the present invention may vary depending on the type of target RNA, and includes, for example, ribosomal protein, snRNP protein, snoRNP protein, and Argonaute-family protein, and among them, Argonaute-family protein is preferable. The Argonaute-family protein includes specifically Argonaute subfamily such as Ago1, Ago2, Ago3, and Ago4, and Piwi subfamily such as PIWIL 1, PIWIL 2, PIWIL 3, PIWIL 4, MILI, and MIWI, however, the Argonaute subfamily such as Ago1, Ago2, Ago3, and Ago4 is preferable, and among them, Ago2 which binds to siRNA and microRNA (miRNA) is particularly preferable.

The small RNA to which the small RNA-binding protein involved in the present invention binds includes 5SrRNA, tRNA, small nuclear RNA (snRNA), small nucleolar RNA (snoRNA), siRNA, piRNA, miRNA and the like; among them, piRNA, siRNA, miRNA and the like are preferable, siRNA, miRNA and the like are more preferable, and miRNA and the like is particularly preferable. In addition, length thereof is usually 5 to 200 nt, preferably 10 to 50 nt, and more preferably 10 to 30 nt.

The physiologically active substance which has affinity to the small RNA-binding protein (hereinafter, optionally abbreviated as physiologically active substance involved in the present invention) may vary depending on the type of the above-described small RNA-binding protein, and includes antibody which has affinity (binding ability) to the small RNA-binding protein, aptamer, and the like; and among them, antibody is preferable. Specifically, for example, when Ago2 is employed as a small RNA-binding protein, anti-Ago2 antibody is used; and when Ago3 is employed, anti-Ago3 antibody is used.

The origin of the antibody to be used as a physiologically active substance involved in the present invention is not particularly limited, and may be either a polyclonal antibody or a monoclonal antibody, but monoclonal antibody is more preferable. In addition, as these antibodies, commercially available antibody products may be employed, and if necessary, $F(ab')_2$, Fab' or Fab, which is prepared by digesting using an enzyme such as pepsin, papain, and the like, may be used.

As to the method for obtaining the above-described polyclonal antibody, the polyclonal antibody may be prepared according to the method described in "Sunao Matsuhashi, et al., Introduction of Experimental Immunology, 2nd printing, Japan Scientific Society Press, 1981", etc., by the common procedure for immunizing an animal such as, for example, horse, cattle, sheep, rabbit, goat, guinea pigs, rat, and mouse with an objective substance to be measured. In addition, the method for obtaining monoclonal antibody includes a method in which an immunologically sensitized cell such as, for example, spleen cell, lymphocyte or the like of an animal such as, for example, rat, mouse or the like which has been immunized against the objective substance to be measured as an immunogen, and a cell which has a property of immortal growth, such as, for example, myeloma cell are fused to produce a hybridoma by the cell fusion technology well known per se developed by Kohler and Milstein (Nature, 256, 495, 1975), then said hybridoma is cultured in a culture medium or injected into the intraperitoneal cavity to produce the antibody, and the objective monoclonal antibody is harvested from said culture medium or ascites fluid, and a method in which the antibody-producing cells having the property as described above are produced by a method well known per se utilizing genetic engineering techniques (Eur. J. Immunol., 6, 511, 1976), and by culturing the cells to obtain the objective monoclonal antibody.

In addition, as to the method for preparing the above-described aptamer, the methods described in US patent No. 5,270,163, etc. is preferable.

Amount of the physiologically active substance involved in the present invention to be immobilized on the carrier for obtaining small RNA of the present invention (hereinafter, optionally abbreviated as carrier of the present invention) may vary depending on the kind of the physiologically active substance employed, and is usually 0.1 to 10 mg, preferably 1 to 10 mg per 1g of the carrier.

The carrier of the present invention is the one in which the physiologically active substance involved in the present invention has been immobilized on the surface of the above-described particle involved in the present invention, and which is used in the method for obtaining small RNA of the present invention. As to the method of immobilizing the physiologically active substance involved in the present invention on the surface of the particle involved in the present invention, a method of immobilization well known per se such as, for example, a method of immobilization by a chemical bond such as a covalent bond or a method of immobilization by physical adsorption (JP-B-5-41946, and the like) may be utilized, however, the method of immobilization by a chemical bond is preferable, and in particular, the method of immobilization by using a functional group which has been introduced on the surface of the particle to immobilize the physiologically active substance is particularly preferable. Specifically, for example, a 0.5 mL of solution containing usually 2 to 200 μg/mL, preferably 20 to 200 μg/mL physiologically active substance involved in the present invention is contacted with 10 mg of the particles involved in the present invention, if necessary, in the coexistence of an appropriate condensation agent and the like, and allowed to react at usually 20 to 50°C, preferably 30 to 40°C for usually 1 to 20 hours, preferably 1 to 10 hours, more preferably 2 to 5 hours, and thereby the carrier of the present invention in which the physiologically active substance has been immobilized on the surface thereof can be obtained. Here, as the condensation agent to be used if necessary, the one which is used in the common procedures in this field may be employed

appropriately. In addition, after the physiologically active substance is immobilized, it is desirable to perform a treatment with blocking agents such as ethanolamine, etc., to inactivate the functional group on the surface of the particle involved in the present invention to which the physiologically active substance has not been immobilized.

The solvent to be used for preparing a solution of the physiologically active substance involved in the present invention may be the one which does not have a property of preventing adsorption or binding of the physiologically active substance to the insoluble carrier, for example, purified water and buffer solution such as, for example, phosphate buffer solution, Tris buffer solution, Good's buffer solution, glycine buffer solution, borate buffer solution, sodium bicarbonate buffer solution and so on, having a buffering action, for example, at pH 5.0 to 10.0, preferably pH 8.5 to 10, are preferable. In addition, concentration of the buffering agent in these buffer solutions is selected as appropriate from the range of usually 0.1 to 5 M, and preferably 0.6 to 2.5 M. In addition, in this solution, as long as it is the amount for example, sugar, salts such as NaCl, surface-active agent, antiseptic agent, protein, and the like may be contained, within an amount which does not interfere said antibody to be adsorbed or bound on the insoluble carrier. It should be noted that, since the particles involved in the present invention exhibit less nonspecific adsorption, the carrier of the present invention obtained by the process as mentioned above is not necessary to be subjected to the blocking process usually performed in this field.

Specifically, the carrier of the present invention is produced as described below.

That is, for example, to an aqueous solution containing 0.01 to 1.0 mol/L of silane coupling agent in acetic acid (for example, pH 2 to 4), 1 to 10 g of silica beads are added, and incubated at 50 to 100°C for 10 to 180 minutes, to produce the silica beads which have a polymerizable functional group or a chain transfer group. On the other hand, the monomer represented by the general formula [1] and the monomer represented by the general formula [2] are mixed in a ratio of 1:99 to 70:30, and dissolved in a solvent such as dehydrated ethanol. Subsequently, 1 to 10 times mol of a polymerization initiator such as AIBN is added to said solution, followed by agitation to obtain a mixed monomer solution. The silica beads are added to said mixed monomer solution so that 0.1 to 10 mmol of the above-described polymer per 1 g of the silica beads which have the above-described polymerizable functional group or chain transfer group exists. The mixture is reacted at 50 to 80°C for 10 to 30 hours, if necessary under argon atmosphere, followed by drying to obtain the particle involved in the present invention. Subsequently, the particle involved in the present invention is added to a sodium bicarbonate buffer solution containing the physiologically active substance involved in the present invention so that 0.1 to 10 mg of the physiologically active substance involved in the present invention such as anti-Ago2 antibody etc. per 1 g of said particle exists. The mixture is reacted at 30 to 40°C for 2 to 10 hours, to immobilize the physiologically active substance involved in the present invention on the surface of the particle, and obtain the carrier of the present invention.

The above-described carrier of the present invention exhibits less nonspecific adsorption, and therefore can specifically adsorb a complex of small RNA-binding protein and small RNA, and further, when small RNA has been bound to target nucleic acid (for example, in the case where the small RNA is miRNA or piRNA), a complex of small RNA-binding protein, small RNA and target nucleic acid of small RNA (hereinafter, these are optionally collectively referred to as small RNA-binding protein complex). If the carrier adsorbs proteins other than the small RNA-binding protein complex nonspecifically, the adsorbed protein may cause to inhibit the small RNA-binding protein complex, etc. to bind to the carrier (steric hindrance inhibition, etc.). Consequently, recovery rate of the small RNA-binding protein complex etc. will be decreased. However, since the carrier of the present invention has less possibility to generate such steric hindrance, by the method employing said carrier, the small RNA-binding protein complexes etc. can be recovered in high yield, and as a result, small RNA etc. can be obtained in high yield as well.

The method for obtaining the small RNA of the present invention is a method for obtaining the small RNA by employing the carrier of the present invention obtained as described above, and specifically comprises the following steps.

Namely, the method comprises step (1): introducing a polymerizable functional group or a chain transfer group to the surface of core particle, mixing said particle with a polymerizable monomer, subsequently promoting polymerization reaction to form a layer comprising a polymer compound on the surface of said particle, and immobilizing a physiologically active substance having affinity to small RNA-binding protein on the surface of said particle to form a carrier, and contacting the carrier with a complex of said small RNA-binding protein and a small RNA (small RNA-binding protein-small RNA complex) to form a bound substance of said physiologically active substance and said small RNA-binding protein-small RNA complex (hereinafter, optionally abbreviated as physiologically active substance- small RNA-binding protein-small RNA bound substance) on the surface of said carrier; step (2): isolating the obtained carrier having physiologically active substance- small RNA-binding protein-small RNA bound substance on the surface thereof; step (3): eluting the small RNA from said bound substance of physiologically active substance and small RNA-binding protein-small RNA complex; and step (4): purifying the eluted small RNA.

Specifically, firstly, the carrier of the present invention is added to a solution containing small RNA-binding protein-small RNA complex, and the mixture is reacted at 2 to 37°C, preferably at 2 to 10°C for 1 to 30 hours, preferably for 2 to 24 hours to form a complex of the physiologically active substance and the small RNA-binding protein-small RNA complex on the surface of the carrier of the present invention (step (1)). The above-described small RNA-binding

protein-small RNA complex is the one which comprises a complex produced by binding the small RNA-binding protein involved in the present invention to the small RNA involved in the present invention, and may further comprise a protein which binds to said complex (for example, Gemin3, Gemin4, FMRP, etc.). Specific example of said complex includes, for example, RISC (RNA-induced silencing complex) and the like. The solution containing the small RNA-binding protein-small RNA complex to be used in this case includes a cell lysate (cell extract) containing the small RNA-binding protein-small RNA complex, and said cell lysate containing usually 5 x $10^6$ to 1 x $10^7$ cells per 1 mL solution is used. The solvent (reaction solvent) of the solution containing the small RNA-binding protein-small RNA complex includes all of the cell-lysing liquid usually used in this field, and specifically, for example, a buffer solution containing surface active agent and NaCl. The surface active agent includes, for example, poly(oxyethylene) nonylphenyl ether (NP-40) and the like, and concentration thereof is usually 0.01 to 0.5% to the total amount of buffer solution. Concentration of said NaCl is usually 100 to 200 mM as the concentration in the buffer solution. In addition, as to the buffer solution, a buffer solution having buffering action under near neutral condition, for example, at pH 5.0 to 10.0, preferably pH 6.5 to 8.5, including, for example, phosphate buffer solution, Tris buffer solution, Good's buffer solution, glycine buffer solution, borate buffer solution and so on, is preferable. In addition, concentration of the buffering agent in the buffer solution is selected appropriately from a range of usually 10 to 500 mM, preferably 10 to 300 mM. Further, to the above-described buffer solution containing surface active agent and NaCl, TritonX-100, SDS, or the like may be added, if necessary, at a concentration usually employed in this field. In the above-described step (1), amount of the carrier of the present invention to be used is usually 1 to 10 mg, preferably I to 5 mg per 1 mL solution of the above-described small RNA-binding protein-small RNA complex, and amount of the physiologically active substance involved in the present invention is usually 0.1 to 100 μg, preferably 1 to 50 μg.

Next, after the reaction of the above-described step (1), the carrier having the physiologically active substance-small RNA-binding protein-small RNA bound substance on the surface thereof is separated from the obtained reaction solution (step (2)). Specifically, after subjecting the reaction solution of step (1) to a centrifugal separation, the supernatant solution is removed, and thereby the carrier of the present invention is separated from the reaction solution of step (1). Furthermore, in order to remove the free small RNA-binding protein-small RNA complex and cell-derived substances attached to the surface of the carrier, it is preferable to wash the obtained carrier with washing liquid. The above-described centrifugal separation is not particularly limited so long as it is carried out in an aspect usually performed in this field, and may be performed by conducting the procedure in which the carrier of the present invention suspended in an appropriate washing liquid is centrifuged, for example, by 1,000 to 10,000 x g for 10 to 100 seconds, preferably repeating this procedure several times. The washing liquid may be a buffer solution usually employed in this field having a buffering action under near neutral condition, for example, at pH 5.0 to 10.0, preferably pH 6.5 to 8.5, including, for example, phosphate buffer solution, Tris buffer solution, Good's buffer solution, glycine buffer solution, borate buffer solution and so on. In addition, concentration of the buffering agent in the buffer solution is selected appropriately from a range of usually 10 to 500 mM, preferably 10 to 300 mM. It should be noted that, for said washing liquid, it is preferable to use the same one as the reaction solvent used in step (1). Amount of the washing liquid is usually 1 to 10 times amount of the reaction solvent used in the above-described step (1).

Subsequently, after separation of the carrier having the above-described bound substance on the surface in the above-described step (2), the small RNA is eluted from the physiologically active substance-small RNA-binding protein-small RNA bound substance on the surface. Specifically, the elution is performed by denaturing the physiologically active substance by adding a protein denaturing agent to the carrier having the above-described bound substance on the surface thereof. Thereby, the physiologically active substance and the small RNA-binding protein are denatured, and the small RNA is set apart and eluted (step (3)). The protein denaturing agent employed here may be any substance which is capable of denaturing the physiologically active substance involved in the present invention, and includes, for example, sodium dodecyl sulfate (SDS), sodium lauryl sulfate, urea, guanidine hydrochlorides, guanidine thiocyanate, citrate buffer solution (pH 2 to 4), and the like, and among them, SDS is preferable. Amount thereof to be used is 10 to 1,000 μL, preferably 10 to 400 μL per 1 to 5 mg of the carrier.

Further, the small RNA is isolated by purifying the small RNA eluted in the above-described step (3) by a nucleic acid extraction method, etc. (step (4)). As to the nucleic acid extraction method, any method may be employed so long as it is the one usually used in this field, and the method includes, for example, extraction with a mixed solution of phenol/chloroform/isoamyl alcohol. Ratio of the above-described mixed solution is usually phenol:chloroform:isoamyl alcohol = 25:24:1, and amount of the mixed solution to be used is the same amount to the elution solution. The small RNA obtained as described above is more preferably purified by the method usually employed in this field, such as alcohol precipitation, column purification, and filtration through a filter, and so on.

The method for obtaining the small RNA of the present invention is performed more specifically as follows. That is, for example, to 1 mL of the cell lysate prepared from 5 x $10^6$ to 1 x $10^7$ cells containing a complex of Ago2 and small RNA, 1 to 10 mg, preferably 1 to 5 mg of the carrier of the present invention is added, and the mixture is reacted at 2 to 10°C for 2 to 24 hours. Further, after carrying out centrifugal separation of the obtained reaction solution by 3,000 to 5,000 x g for 10 to 50 seconds and removing the supernatant solution, the carrier to which the complex has been

bound is washed several times using 1 to 5 mL of Tris buffer solution (pH 6.5 to 8.5) per 1 mL of the reaction solution. Subsequently, 10 to 400 μL of SDS solution is added per 1 to 5 mg of the carrier of the present invention, and the small RNA is eluted from the carrier. Further, after the small RNA is extracted by adding 1 μL of phenol/chloroform/isoamyl alcohol (25:24:1) per 1 μL of the eluate solution, 2 to 3 μL of ethanol, 0.05 to 0.1 μL of sodium acetate, 0.005 to 0.01 μL of coprecipitating agent such as Ethachinmate, glycogen, and the like are added per 1 μL of extraction solution, and thereby the purified small RNA is obtained as precipitate.

In the method for obtaining a small RNA of the present invention, since the carrier to which a physiologically active substance has been immobilized on the surface (the carrier of the present invention) is utilized, as compared with the conventional methods, the step in which the physiologically active substance such as antibody is immobilized to the carrier can be skipped. Also, addition of a blocking agent and the like for preventing nonspecific adsorption is not necessary; the purification procedure is also simple; and therefore small RNA can be obtained by simple operation.

Some small RNA binds to nucleic acid as a target. Utilizing such property, by using the carrier of the present invention, it may also become possible to obtain the target nucleic acid of the small RNA. Here, the target nucleic acid may be either DNA or RNA, as long as it is nucleic acid which is targeted by a small RNA, and the chain length thereof is also not particularly limited. The target mentioned here means that the small RNA has an affinity thereto, specifically, preferably binds thereto in a complementary style. When the small RNA binds in a complementary style, whole chain is not necessary to be complementary, but at least six or more bases of the small RNA and the target nucleic acid need to be complementary. As a target nucleic acid, for example, when the small RNA is microRNA, RNA is preferable and mRNA is more preferable. In addition, when the small RNA is piRNA, RNA is preferable, but when DNA can serve as a target nucleic acid, it may be DNA.

Method for obtaining a target nucleic acid of small RNA is carried out as follows. Namely, since the small RNA having the above-described properties is present in combination with target nucleic acid in a cell, bound substance of small RNA and target nucleic acid thereof may be obtained by performing steps (1), (2) and (3), preferably steps (1), (2), (3), and (4) of the above-described method for obtaining small RNA of the present invention. Therefore, the bound substance of the small RNA and target nucleic acid thereof is dissociated by adding a solution such as formamide, subjecting the bound substance, for example, to denatured acrylamide gel electrophoresis, and extracting the nucleic acid having the desired nucleotide (nt), to obtain the nucleic acid which is targeted by the small RNA. Concentration of the target nucleic acid of small RNA thus obtained is usually low. Therefore, when the target nucleic acid of small RNA is DNA, the obtained nucleic acid may be subjected to the PCR reaction to be amplified. Since the concentration of the target nucleic acid in the bound substance of the small RNA and the target nucleic acid thereof obtained as described above is also low, said bound substance may be subjected directly to the PCR reaction to be amplified. In this connection, condition of the PCR reaction is set up according to the conditions usually carried out in this field.

In addition, when the target nucleic acid is RNA, the bound substance of small RNA and the target nucleic acid thereof obtained as mentioned above is subjected to the reverse transcription reaction using a primer corresponding to the target nucleic acid to obtain cDNA of the target nucleic acid, which is subjected to the PCR reaction in the same way as the case of the above-described DNA to be amplified. Here, conditions of reverse transcription reaction are set up according to the conditions usually carried out in this field. In addition, by sequencing the DNA obtained as described above, it becomes possible to identify the target nucleic acid of the small RNA. Furthermore, analysis of actions, such as cleavage and suppression of translation of the target nucleic acid by the small RNA-binding protein-small RNA complex etc. also becomes possible.

Hereinafter, the present invention will be explained in detail by referring to Examples and Comparative Examples.

## Examples

**<u>Experimental Example 1:</u>** <u>Preparation of mouse IgG-immobilized carrier using the particle involved in the present invention</u>

(1) Preparation of a particle (bead) for protein immobilization

[Synthesis of p-nitrophenyl oxycarbonyl-polyethylene glycol methacrylate (MEONP)]

After dissolving 0.01 mol of polyethylene glycol monomethacrylate (Blenmer PE-200, Nippon Oil & Fats Co., Ltd.) in 20 mL of chloroform, the solution was cooled down to -30°C. To this solution, a homogeneous solution of 0.01 mol of p-nitrophenyl chloroformate (Aldrich Chemical Co., Inc.), 0.01 mol of triethylamine (Wako Pure Chemicals Industries Ltd.) and 20 ml of chloroform which had been prepared in advance was added drop-wise slowly, while the solution was kept at -30°C. After reacting at -30°C for 1 hour, the solution was stirred at room temperatures for another 2 hours. Then, the salt was removed from the reaction solution by filtration and solvent was distilled off, to obtain a crude material of p-nitrophenyl oxycarbonyl-polyethylene glycol methacrylate (hereinafter, referred to as MEONP). Furthermore, the

obtained crude material was purified using silica gel column. $^1H$ NMR of the obtained monomer was measured in deuterated chloroform, and it was confirmed that 4.5 units of ethylene glycol residue were contained.

[Preparation of silica beads treated with methacryloxypropyl trimethoxysilane]

To 39.3 g of aqueous acetic acid solution of pH 3.0, 7.45 g of methacryloxypropyl trimethoxysilane (LS3380, produced by Shin-Etsu Chemical Co., Ltd.) was added, and the solution was stirred at room temperatures for 1 hour. To this solution, 5 g of silica beads (SMB70-5; produced by Fuji Silysia Chemical Ltd.; average particle size: 5 μm; pore size: 70 angstrom) was added, and the solution was stirred at 85°C for 2 hours, then the silica beads were recovered from the reaction solution by suction filtration and heated at 100°C for 1 hour. After that, procedures of dispersion of the beads in ethanol, shaking at room temperatures for 1 hour, then centrifugation and removal of supernatant solution were repeated twice. Furthermore, procedures of dispersion of the beads in ethanol, agitation by voltex mixer, then centrifugation and removal of the supernatant solution were repeated 5 times, and then the beads were dried.

[Preparation of a particle (bead) for protein immobilization]

Poly(ethylene glycol) methyl ether methacrylate of number average molecular weight Mn about 475 (alias: methoxypolyethylene glycol methacrylate, hereinafter referred to as PEGMA475; produced by Aldrich Chemical Co., Inc.) and MEONP obtained as described above were dissolved in absolute ethanol, to prepare a mixed monomer solution. The total monomer concentration was set to 0.2 mol/L and molar ratio of the monomers was set to PEGMA475:MEONP = 80:20. To this mixed solution, azobisisobutyronitryl (AIBN) was added so as to become 0.004 mol/L, and the solution was stirred until it became homogeneous. Then, 1 g of the silica beads which had been treated with the above-described methacryloxypropyl trimethoxysilane was added, and the solution was reacted under argon atmosphere at 60°C for 22 hours. Subsequently, the silica beads were recovered from the reaction solution by suction filtration, and procedures of dispersion in ethanol, then centrifugation and removal of the supernatant solution were repeated 5 times. After that, the beads were recovered by suction filtration and dried well.

(2) Preparation of mouse IgG-immobilized carrier

[Preparation of mouse IgG solution]

In 1 mL of phosphate buffer solution (PBS, pH 7.4, produced by Wako Pure Chemical Industries Ltd.), 1 mg of mouse IgG (produced by Wako Pure Chemical Industries Ltd.) was dissolved, and this solution was used as a 1 mg/mL mouseIgG solution.

[Preparation of mouse IgG-immobilized carrier]

A 10 mg of the particle for protein immobilization obtained in the above-described (1), 50 μL of mouse IgG solution, and 500 μL of buffer solution for protein immobilization (produced by Sumitomo Bakelite Company, Ltd.) were mixed by inversion at 37°C for 4 hours. After centrifugal separation (3,000 x g for 1 minute), the supernatant solution was removed using pipette, and then the particle was washed twice with 500 μL of PBS (pH 7.4). After that, 500 μL of the buffer solution for inactivation (produced by Sumitomo Bakelite Company., Ltd.) was added and mixed by inversion at room temperatures for 1 hour. After centrifugal separation (3,000 x g for I minute), the supernatant solution was removed using pipette, and then the particle was washed 5 times with 500 μL of PBS (pH 7.4), and suspended in 1 mL of PBS (pH 7.4).

**Comparative Example 1:** Preparation of mouse IgG antibody-immobilized carrier using various types of carriers

(1) Immobilization of mouse IgG to the carrier produced by GE Healthcare Company

Using NHS-activated Sepharose 4 Fast Flow (produced by GE Healthcare Company) as a carrier, and according to the protocol recommended by the company, the mouse IgG antibody was immobilized as follows. That is, after washing 100 μL of NHS-activated Sepharose 4 Fast Flow twice with 500 μL of ice cooled 1 mM hydrochloric acid, 50 μL of mouse IgG solution and 500 μL of coupling buffer solution (0.5 M sodium chloride containing 0.2 M sodium bicarbonate, pH 8.3) were added, and the mixture was mixed by inversion at room temperature for 4 hours. After centrifugal separation (3,000 x g for 1 minute), the supernatant solution was removed using pipette, and then the particle was washed with 500 μL of coupling buffer solution. After that, 500 μL of quenching buffer solution (0.5 M ethanolamine, 0.5 M sodium chloride (pH 8.3)) was added and the mixture was mixed by inversion at room temperature for 30 minutes. After centrifugal

separation (3,000 x g for 1 minute), the supernatant solution was removed using pipette, and then the particle was washed 3 times with 500 μL of quenching buffer solution and 500 μL of washing liquid (0.5 M sodium chloride containing 0.1 M acetic acid, pH 4) alternately, then washed 3 times with 500 μL of PBS (pH 7.4), and then suspended in 1 mL of PBS (pH 7.4).

(2) Immobilization of mouse IgG to the carrier produced by Pierce Biotechnology Inc.

Using an immunoprecipitation kit (Seize-Primary Mammalian Immunoprecipitation Kit, produced by Pierce Biotechnology Inc.), and according to the protocol recommended by the company, mouse IgG (produced by Wako Pure Chemical Industries Ltd.) was immobilized. After washing 100 μL of beads of the kit component twice with 400 μL of coupling buffer (produced by Pierce Biotechnology Inc.), 50 μL of mouse IgG solution, 200 μL of coupling buffer solution, and 4 μL of 5 M sodium cyanoborohydride (produced by Pierce Biotechnology Inc.) were added, and the mixture was mixed by inversion at room temperature for 4 hours. After centrifugal separation (3,000 x g for 1 minute), the supernatant solution was removed using pipette, and the particle was washed with 400 μL of coupling buffer solution, then, 400 μL of quenching buffer solution (produced by Pierce Biotechnology Inc.) and 4 μL of 5 M sodium cyanoborohydride were added and the mixture was mixed by inversion at room temperature for 30 minutes. After centrifugal separation (3,000 x g for 1 minute), the supernatant solution was removed using pipette, and then the particle was washed 6 times with 400 μL of washing liquid (produced by Pierce Biotechnology Inc.), then washed 3 times with 400 μL of PBS (pH 7.4), and suspended in 1 mL of PBS (pH 7.4).

(3) Immobilization of mouse IgG to the carrier produced by Dynal Biotech.

Using Dynabeads M-270 Carboxylic Acid (produced by Dynal Biotech.) as a carrier, and according to the protocol recommended by the company, the mouse IgG antibody was immobilized as follows. After washing 100 μL of Dynabeads M-270 Carboxylic Acid 3 times with 500 μL of reaction buffer solution (0.1 M MES, pH 5.0), 50 μL of mouse IgG solution and 500 μL of reaction buffer solution were added, and the mixture was mixed by inversion at room temperature for 30 minutes. After that, 25 μL of 10 mg/mL water soluble carbodiimide (WSC) solution was added, and the mixture was mixed by inversion at room temperature overnight. After magnetic separation, the beads were washed 4 times with 500 μL of washing liquid (0.05w/v% Tween20 containing 25 mM Tris-HCl and 150 mM sodium chloride (pH 7.4)), and suspended in 1 mL of PBS (pH 7.4).

**Experimental Example 2:** Experiment on nonspecific adsorption of mouse IgG antibody-immobilized carriers prepared using various types of carriers

[Preparation of cell lysate]

HeLa cell (produced by Dainippon Pharmaceutical Co., Ltd.) was cultured in Dullbecco's Modified Eagle's Medium (DMEM, produced by Wako Pure Chemical Industries Ltd.) which was supplemented with fetal bovin serum (FBS, produced by Thermo Electron Corporation) so that final concentration became 10 v/v%, and after removing the cultured medium, cells were washed twice with PBS (-) (calcium and magnesium free PBS). After detaching the cells from a dish using trypsin-EDTA solution (produced by Wako Pure Chemical Industries Ltd.), the culture medium was added to quench trypsin activity, then the culture solution was centrifuged (1,000 x g for 5 minutes) and the supernatant solution was removed. Cell pellet was suspended in 1 mL of PBS(-) and number of cells was counted; 1 x $10^7$ cells were transferred into the sterilized 1.5 mL tube and centrifuged (1,000 x g for 5 minutes), and the supernatant solution was removed.

To the cell pellet, 1 mL of cell lysis solution (0.05w/v% NP-40 (produced by Wako Pure Chemical Industries Ltd.) containing 20 mM Tris-HCl, 200 mM sodium chloride, and 2.5 mM magnesium chloride) was added, and the cells were suspended by pipetting and the suspension was allowed to stand on ice for 10 minutes. After centrifugal separation (20,000 x g for 20 minutes, at 4°C), the supernatant solution (cell lysate) was isolated.

[Test for nonspecific adsorption of cell-derived protein]

Each 200 μL of the mouse IgG-immobilized carrier of the present invention obtained in Experimental Example 1 and the mouse IgG-immobilized carriers of various manufacturers obtained in Comparative Example 1 were centrifuged (3,000 x g for 30 seconds) or magnetically separated, and respective supernatant solutions were removed, and then 1 mL of the cell lysate was added to each carrier and the mixture was mixed by inversion in refrigerator overnight. After centrifugal separation (3,000 x g for 30 seconds) or magnetic separation, the supernatant solutions were removed, and the carriers were washed 3 times with 1 mL of the cell lysis solution. After that, 20 μL of 2w/v% sodium dodecyl sulfate

(SDS) solution was added on the pellet, and the protein adsorbed on the carrier was eluted.

To 10 μL of the eluate, 2 x SDS sample buffer solution (produced by Wako Pure Chemical Industries Ltd.) was added, and incubated at 95°C for 3 minutes. The resultant sample was applied to Super Sep HG10-20 w/v% gel (produced by Wako Pure Chemical Industries Ltd.), and SDS polyacrylamide gel electrophoresis (25mA, 1 hour) was carried out. After that, the gel was silver stained using Silver Stain 2 Kit wako (produced by Wako Pure Chemical Industries Ltd.)

Results of the SDS polyacrylamide gel electrophoresis are shown in Fig. 1.

As is clear from the results shown in Fig. 1, it can be found out that nonspecific adsorption of cell-derived protein by the mouse IgG-immobilized carrier employing the particle involved in the present invention is low compared with the carrier from each manufacturer in which mouse IgG is immobilized obtained in Comparative Example 1.

**Example 1:** Preparation of anti-human Ago2 antibody-immobilized carrier

To 50 μL of 1 mg/mL anti-human Ago2 antibody (produced by Wako Pure Chemical Industries Ltd.), 10mg of particles for protein immobilization obtained in Experimental Example 1 (1), 500 μL of buffer solution for protein immobilization (produced by Sumitomo Bakelite Company, Ltd.) were mixed and the mixture was mixed by inversion at 37°C for 4 hours. After centrifugal separation (3,000 x g for 1 minute), the supernatant solution was removed using pipette, and then the particle was washed twice with 500 μL of PBS (pH 7.4). After that, 500 μL of buffer solution for inactivation (produced by Sumitomo Bakelite Company., Ltd.) was added and the mixture was mixed by inversion at room temperatures for 1 hour. After centrifugal separation (3,000 x g for 1 minute), the supernatant solution was removed using pipette, and then the particle was washed 5 times with 500 μL of PBS (pH 7.4), and suspended in 1 mL of PBS (pH 7.4), to obtain anti-human Ago2 antibody-immobilized carrier.

**Comparative Example 2:** Preparation of anti-human Ago2 antibody-immobilized carrier using various types of carriers

(1) Immobilization of anti-human Ago2 antibody to the carrier produced by GE Healthcare Company

Anti-human Ago2 antibody was immobilized to the carrier by the same procedures as in the immobilization of mouse IgG to the carrier produced by GE Health Care Company in Comparative Example 1 (1), excepting that 50 μL of 1 mg/mL anti-human Ago2 antibody (produced by Wako Pure Chemical Industries Ltd.) was used instead of 50 μL of mouse IgG solution. (2) Immobilization of anti-human Ago2 antibody to the carrier produced by Pierce Biotechnology Inc.

Anti-human Ago2 antibody was immobilized to the carrier by the same procedures as in the immobilization of mouse IgG to the carrier produced by Pierce Biotechnology Inc. in Comparative Example 1 (2), excepting that 50 μL of 1 mg/mL anti-human Ago2 antibody (produced by Wako Pure Chemical Industries Ltd.) was used instead of 50 μL of mouse IgG solution. (3) Immobilization of anti-human Ago2 antibody to the carrier produced by Dynal Biotech.

Anti-human Ago2 antibody was immobilized to the carrier by the same procedures as in the immobilization of mouse IgG to the carrier produced by Dynal Biotech. in Comparative Example 1 (3), excepting that 50 μL of 1 mg/mL anti-human Ago2 antibody (produced by Wako Pure Chemical Industries Ltd.) was used instead of 50 μL of mouse IgG solution.

**Experimental Example 3:** Experiment on specific adsorption of anti-human Ago2 antibody-immobilized carriers prepared using various types of carriers

[Test for specific adsorption of cell-derived protein]

Each 200 μL of the anti-human Ago2 antibody-immobilized carrier obtained in Example 1 and the anti-human Ago2 antibody-immobilized carrier from various manufacturers obtained in Comparative Example 2 were centrifuged (3,000 x g for 30 seconds) or magnetically separated, and respective supernatant solutions were removed, and then 1 mL of the cell lysate obtained in Experimental Example 2 was added to each carrier and the mixture was mixed by inversion in refrigerator overnight. After centrifugal separation (3,000 x g for 30 seconds) or magnetic separation, the supernatant solutions were removed, and the carriers were washed 3 times with 1 mL of the cell lysis solution. After that, 20 μL of 2 w/v% sodium dodecyl sulfate (SDS) solution was added on the pellet, and the protein adsorbed on the carrier was eluted.

To 10 μL of each eluate, 10 μL of 2 x SDS sample buffer solution (produced by Wako Pure Chemical Industries Ltd.) was added, and the solution was incubated at 95°C for 3 minutes. The resultant sample was applied to Super Sep HG10-20 w/v% gel (produced by Wako Pure Chemical Industries Ltd.), and SDS polyacrylamide gel electrophoresis (25 mA, 1 hour) was carried out. After that, the gel was silver stained using Silver Stain 2 Kit Wako (produced by Wako Pure Chemical Industries Ltd.).

Results of SDS polyacrylamide gel electrophoresis are shown in Fig. 2.

As is clear from the results shown in Fig. 2, it can be found out that in the case of the anti-human Ago2 antibody-immobilized carrier in which the particle involved in the present invention is employed, a band at about 100 kDa corresponding to Ago2 is observed, while it is not seen in Fig. 1, indicating that the Ago2 is trapped on the carrier specifically. Moreover, it can also be found out that, in comparison with the results obtained for various types of carriers in which the anti-human Ago2 antibodies obtained in Comparative Example 2 are immobilized, when the carrier of the present invention is employed, the band corresponding to Ago2 appears deeply, but generally number of nonspecifically adsorbed cell-derived protein bands is less, and amount of nonspecifically adsorbed substances other than Ago2 is low. That is, it can be understand that, when said carrier is employed, Ago2 and miRNA coupled with Ago2 can be obtained in high yield.

**Example 2:** Method for isolating miRNA

[Preparation of a solution for immobilizing anti-human Ago2 antibody]

To 25, 50, and 100 μL of 1 mg/mL anti-human Ago2 antibodies (produced by Wako Pure Chemical Industries Ltd.), 475, 450, and 400 μL of buffer solutions for protein immobilization (produced by Sumitomo Bakelite Co., Ltd.) were added respectively and each solution was mixed, and 2.5, 5, and 10 μg antibody/mg bead solutions for immobilization were prepared.

[Preparation of anti-human Ago2 antibody-immobilized carrier]

To 10 mg of particle for protein immobilization obtained in Experimental Example 1 (1), each 500 μL of the above-prepared various types of anti-human Ago2 antibody immobilization solutions was mixed, and the mixture was mixed by inversion at 37°C for 4 hours. After centrifugal separation (3,000 x g for 1 minute), the supernatant solution was removed using pipette, and then the particle was washed twice with 500 μL of PBS (pH 7.4). After that, 500 μL of the buffer solution for inactivation (produced by Sumitomo Bakelite Company., Ltd.) was added and the mixture was mixed by inversion at room temperatures for 1 hour. After centrifugal separation (3,000 x g for 1 minute), the supernatant solution was removed using pipette, and then the particle was washed 5 times with 500 μL of PBS (pH 7.4), and suspended in 1 mL of PBS (pH 7.4).

[Preparation of cell lysate]

HeLa cell (produced by Dainippon Pharmaceutical Co., Ltd.) was cultured in DMEM (produced by Wako Pure Chemical Industries Ltd.) which was supplemented with FBS (produced by Thermo Electron Corporation) so that final concentration became 10v/v%, and after removal of the cultured medium, cells were washed twice with PBS (-). After detaching the cells from the dish using trypsin-EDTA solution (produced by Wako Pure Chemical Industries Ltd.), the culture medium was added to quench trypsin activity, then the culture solution was centrifuged (1,000 x g for 5 minutes) and the supernatant solution was removed. The cell pellet was suspended in 1 mL of PBS(-) and number of cells was counted, and 1 x $10^7$ cells were transferred into a sterilized 1.5 mL tube and centrifuged (1,000 x g for 5 minutes), and the supernatant solution was removed.

To the cell pellet, 1 mL of cell lysis solution (20 mM Tris-HCl, 200 mM sodium chloride, 2.5 mM magnesium chloride, 0.05w/v% NP-40) was added, and the cells were dispersed by pipetting and the dispersion was allowed to stand on ice for 10 minutes. After centrifugal separation (20,000 x g for 20 minutes, at 4°C), the supernatant solution (cell lysate) was isolated.

[Isolation of cell-derived microRNA (miRNA) by immunoprecipitation method]

After centrifugal separation (3,000 x g for 30 seconds) of 200 μL of anti-human Ago2 antibody-immobilized carrier solution (corresponding to 2 mg of beads) obtained by the method described above, the supernatant solution was removed, then 1 mL of the cell lysate was added thereto, and the mixture was mixed by inversion in a refrigerator overnight. After centrifugal separation (3,000 x g for 30 seconds), the supernatant solution was removed, and the carrier was washed 3 times with 1 mL of the cell lysis solution. After that, 40 μL of 2 w/v% SDS solution was added on the pellet, and the protein adsorbed on the carrier was eluted. To the eluate solution, 360 μL of sterilized water, and 400 μL of phenol/chloroform/isoamyl alcohol (25:24:1) were added, and the solution was mixed by vortex mixer, then centrifuged (20,000 x g, 10 minutes). The upper layer was isolated, and 400μL of chloroform was added thereto. The solution was mixed by vortex mixer, then centrifuged (20,000 x g, 10 minutes). The upper layer was isolated, and 3 μL of Ethachinmate (produced by Nippon Gene Co., Ltd.), 40 μL of 3 M sodium acetate, 1 mL of ethanol were added thereto, and the solution was mixed by vortex mixer, then centrifuged (20,000 x g, 15 minutes). The precipitate was washed with

1 mL of 70 v/v% ethanol, and air dried at room temperature for 20 minutes, then dissolved in 10 μL of sterilized water. The resultant solution was used as a sample for electrophoresis.

The denaturing polyacrylamide gel for electrophoresis was prepared as follows. Firstly, after 1 mL of 5 x TBE (produced by Nippon Gene Co., Ltd.), 4.8 g of urea, 2.5 mL of 40% polyacrylamide solution (acrylamide: methylenebisacrylamide = 19:1), and 2.5 mL of sterilized water were mixed, 10 μL of TEMED (N, N, N, N-tetramethylethylenediamine) and 80 μL of 10% ammonium persulfate were added to cross-link the gel.

To 5 μL of the above-described sample for electrophoresis, 10 μL of formamide (produced by Wako Pure Chemical Industries Ltd.) was added, and after incubation at 80°C for 3 minute, the mixture was cooled rapidly on ice and applied to the above-described denaturing polyacrylamide gel (10 w/v% acrylamide / 8 M urea / 0.5 x TBE). In addition, as a control, to 1 μL each of 0.25 ng/μL, 0.5 ng/μL, 1 ng/μL, and 2 ng/μL synthetic oligo-RNAs having chain length of 22 nucleotides (UAGCUUAUCAGACUGAUGUUGA), 10 μL of formamide was added, and after incubation at 80°C for 3 minute, each mixture was cooled rapidly on ice and applied to the above-described denaturing polyacrylamide gel.

After carrying out electrophoresis (10 mA, 1 hour) in 0.5 x TBE buffer solution, the gel was silver stained using CLEAR STAIN Ag (produced by Nippon Gene Co., Ltd.). Results of the denaturing polyacrylamide gel electrophoresis are shown in Fig. 3.

As is clear from the results shown in Fig. 3, it can be found out that the miRNA fraction can be isolated in high purity by immunoprecipitation method employing the carrier of the present invention.

**Example 3:** Method for isolating the target mRNA corresponding to miRNA

[Preparation of a solution for immobilizing mouse IgG]

In 1 mL of PBS (pH 7.4), 1 mg of mouse IgG (produced by Wako Pure Chemical Industries Ltd.) was dissolved, and the solution was used as 1 mg/mL mouse IgG solution. To 50 μL of 1 mg/mL mouse IgG solution, 450 μL of buffer solution for protein immobilization (produced by Sumitomo Bakelite Co., Ltd.) was added, thereby 5 μg mouse IgG/mg solution for immobilization was prepared.

[Preparation of a solution for immobilizing anti-human Ago2 antibody]

To 50 μL of 1 mg/mL anti-human Ago2 antibody (produced by Wako Pure Chemical Industries Ltd.), 450 μL of buffer solution for protein immobilization (produced by Sumitomo Bakelite Co., Ltd.) was added, and the solution was mixed, to prepare a solution for immobilizing 5 μg anti-human Ago2 antibody to bead.

[Preparation of mouse IgG-immobilized carrier solution and anti-human Ago2 antibody-immobolized carrier solution]

To 10 mg of the particle for protein immobilization obtained in Experimental Example 1 (1), the mouse IgG immobilization solution or the anti-human Ago2 antibody immobilization solution prepared as described above were mixed respectively, and each mixture was mixed by inversion at 37°C for 4 hours. After centrifugal separation (3,000 x g for 1 minute), the supernatant solution was removed using pipette, and then the particle was washed twice with 500 μL of PBS (pH 7.4). After that, 500 μL of the buffer solution for inactivation (produced by Sumitomo Bakelite Company., Ltd.) was added and the mixture was mixed by inversion at room temperatures for 1 hour. After centrifugal separation (3,000 x g for 1 minute), the supernatant solution was removed using pipette, and then the particle was washed 5 times with 500 μL of PBS (pH 7.4), and suspended in 1 mL of the PBS (pH 7.4). The solution containing the carrier with immobilized mouse IgG was used as mouse IgG-immobilized carrier solution, and the solution containing the carrier with immobilized anti-human Ago2 antibody was used as an anti-human Ago2 antibody-immobilized carrier solution.

[Purification of HeLa cell-derived RNA by immunoprecipitation method]

After centrifugal separation (3,000 x g for 30 seconds) of 200 μL each of mouse IgG-immobilized carrier solution and anti-human Ago2 antibody-immobilized carrier solution (corresponding to 2 mg of beads), the supernatant solution was removed, then I mL of the HeLa cell lysate obtained by the same way as in Example 1 was added thereto, and the mixture was mixed by inversion in a refrigerator for 3 hours. After centrifugal separation (3,000 x g for 30 seconds), the supernatant solution was removed, and the carrier was washed 3 times with 1 mL of the cell lysis solution. After that, 50 μL of 0.5w/v% SDS solution was added on the bead pellet, and the protein adsorbed on the carrier was eluted. To the eluate solution, 350 μL of sterilized water, and 400 μL of phenol/chloroform/isoamyl alcohol (25:24:1) were added and the solution was mixed by vortex mixer, then centrifuged (20,000 x g, 10 minutes). The upper layer was isolated, and 400 μL of chloroform was added thereto. The resultant solution was mixed by vortex mixer, then centrifuged (20,000

x g, 10 minutes). The upper layer was isolated, and 3 μL of Ethachinmate (produced by Nippon Gene Co., Ltd.), 40 μL of 3 M sodium acetate, 1 mL of ethanol were added thereto, and the solution was mixed by vortex mixer, then centrifuged (20,000 x g, 15 minutes). The precipitate was washed with 1 mL of 70v/v% ethanol, and air dried at room temperature for 20 minutes, then dissolved in 10 μL of sterilized water, to obtain purified RNAs, respectively. The solution containing the RNA obtained using mouse IgG-immobilized carrier was used as an RNA solution provided by mouse IgG-immobilized carrier, and the solution containing the RNA obtained using anti-human Ago2 antibody-immobilized carrier was used as an RNA solution provided by anti-human Ago2 antibody-immobilized carrier.

[Purification of HeLa cell derived Total RNA]

To the HeLa cell pellet (1 x $10^7$ cells), 1 mL of ISOGEN (produced by Nippon Gene Co., Ltd.) was added, and the cells were lysed by pipetting. Then, 0.2 mL of chloroform was added and the mixture was mixed with vortex mixer. After standing on ice for 1 hour, the mixture was subjected to centrifugal separation (20,000 x g, 10 minutes), and the upper layer was isolated. To the isolated solution, 0.6 mL of chloroform was added and the solution was mixed with vortex mixer, and centrifuged (20,000 x g, 10 minutes). The upper layer was isolated, and 0.6 mL of isopropanol was added thereto. The solution was mixed, and centrifuged (20,000 x g, 10 minutes). The precipitate was washed with 1 mL of 70v/v% ethanol, and air dried at room temperature for 20 minutes, then dissolved in 261 μL of sterilized water, to obtain 1 mg/mL RNA solution.

[Reverse transcription reaction of purified RNA]

To 10 μL each of the RNA solution provided by mouse IgG-immobilized carrier and the RNA solution provided by anti-human Ago2 antibody-immobilized carrier, both obtained by the above-described immunoprecipitation method, 1 μL of sterilized water and 1 μL of 5 mM Oligo-d$(T)_{12-18}$ primer (produced by GE Healthcare Co. Ltd.) were added, respectively, and mixed. In addition, to 1 μL of the above-described purified Total RNA, 1 μL of sterilized water and 1 μL of 5 mM Oligo-d$(T)_{12-18}$ primer (produced by GE Healthcare Co. Ltd.) were added, and mixed. After incubation at 70°C for 3 minutes, each mixed solution was cooled on ice, and 4 μL of 2.5 mM dNTP mixed solution (produced by Nippon Gene Co., Ltd.), 1 μL of RNase Inhibitor Super (produced by Wako Pure Chemical Industries Ltd.), 2 μL of 10 x reaction buffer solution for Reversecript IV (produced by Wako Pure Chemical Industries Ltd.) and 1 μL of Reversecript IV (reverse transcriptase, produced by Wako Pure Chemical Industries Ltd.) were added thereto, and total volume 20 μL of reaction solution was incubated at 42°C for 30 minutes.

[Amplification of Human NRAS cDNA by PCR]

As a primer for PCR amplification of cDNA of human NRAS (GenBank Accession: NM_002524), a forward primer (AATAATAGCAAGTCATTTGCGG) and a reverse primer (CCACACATGGCAATCCCATA) were synthesized (produced by Sigma-Aldrich Japan K.K.).

To 1 μL of each of the above-described reverse transcription reaction products, 1 μL of 5 μM forward primer, 1 μL of 5 μM reverse primer, 0.8 μL of 2.5 mM dNTP mixed solution (produced by Takara Biotech Co., Ltd.), 1 μL of 10 x reaction buffer solution for Ex Taq polymerase (produced by Takara Biotech Co., Ltd.), 0.2 μL of Ex Taq polymerase (produced by Takara Biotech Co., Ltd.) and 5 μL of sterilized water were added, to prepare total 10 μL of reaction solution, then PCR reactions (95°C for 30 seconds, 60°C for 30 seconds, 60°C for 60 seconds) of 40 cycles were carried out.

The sample prepared by adding 1 μL of loading buffer (produced by Nippon Gene Co., Ltd.) to 5 μL of the reaction mixture was applied to a 1.5% agarose gel which was added with ethidium bromide so that final concentration became 1 μg/mL, and electrophoresis (100 V, 25 minutes) in 1 x TAE buffer solution (produced by Nippon Gene Co., Ltd.) was carried out. After electrophoresis was carried out, band on the gel was detected using UV Transilluminator FAS III (produced by Toyobo Co., Ltd.).

Results of the agarose gel electrophoresis are shown in Fig. 4.

NRAS mRNA has been reported as a target mRNA corresponding to let-7 miRNA expressed in a HeLa cell by Johnson et.al, Cell, Vol.120, 635-647, 2005, etc. In the results of Fig. 4, although the amplified band (302bp) of cDNA corresponding to NRAS mRNA was not detected by immunoprecipitation method employing the particle involved in the present invention in which mouse IgG was immobilized, the band was detected by immunoprecipitation method employing the particle involved in the present invention in which anti-Ago2 antibody was immobilized. That is, it can be found out that if the anti-Ago2 antibody-immobilized carrier of the present invention is employed, the target mRNA of miRNA coupled with Ago2 can also be obtained. Moreover, similarly to the result of Experimental Example 2, since the amplified band is not detected in the immunoprecipitation method employing the mouse IgG-immobilized particle involved in the present invention, it can be found out that there is no nonspecific adsorption of target mRNA to the carrier. Therefore,

it becomes evident that according to the immunoprecipitation method employing the anti-Ago2 antibody-immobilized carrier of the present invention, not only miRNA but also mRNA which is a target of miRNA can be obtained.

**Example 4:** Analysis of the target mRNA corresponding to miRNA

Using HepG2 cell (hepatoma cell) as a cell which does not have miR-122 (micro RNA), and introducing miR-122 into said cell, it was examined using the anti-human Ago2 antibody-immobilized carrier whether the target mRNA (AlodoA and CAT-1) of miR-122 can be obtained from the introduced cell.

[Preparation of miR-122 introduced HepG2 cell and firefly luciferase siRNA (GL3) introduced HepG2 cell]

After HepG2 cells (produced by Dainippon Pharmaceutical Co., Ltd.) were cultured in Dullbecco's Modified Eagle's Medium (DMEM, produced by Wako Pure Chemical Industries Ltd.) to which fetal bovin serum (FBS, produced by Thermo Electron Corporation) was added so that final concentration became 10v/v%, and the culture medium was removed, cells were washed twice with PBS (-) (calcium and magnesium free PBS). After detaching the cells from the dish using trypsin-EDTA solution (produced by Wako Pure Chemical Industries Ltd.), a flesh culture medium was added to stop trypsin activity, then the culture solution was centrifuged (1,000 x g for 5 minutes), and the supernatant solution was removed. The cell pellet was suspended in the DMEM medium to which fetal bovin serum was added so that final concentration of became 10v/v%, and number of cels was counted. Into a 210 $cm^2$ culture flask (produced by Corning, Inc.), 5 x $10^6$ cells were transferred together with 50 mL of the DMEM medium to which fetal bovin serum was added so that final concentration of became 10v/v%.

To 10 mL of Opti-MEM culture medium (produced by Invitrogen Japan KK.), 600 pmol hsa-miR-122 synthetic double-stranded RNA (5'-UGGAGUGUGACAAUGGUGUUUGU-3', 5'-AAACACCAUUGUCACACUCCAUA-3') or 600 pmol firefly luciferase siRNA (GL3, produced by Nippon Gene Co., Ltd.) was mixed separately. To each of 2 sorts of the resultant mixed-solutions, 100 μL of Lipofectamine RNAi max (produced by Invitrogen Corporation) was added and mixed. After leaving to stand at room temperature for 20 minutes, the mixture was added to the above-described culture flask and cultured at 37°C for 24 hours.

After removing culture medium from each flask, the flasks were washed twice with PBS (-). Further, after detaching the cells from the dish using trypsin-EDTA solution, the DMEM culture medium to which fetal bovin serum was added so that final concentration became 10 v/v% was added to suppress trypsin activity, then the culture solution was centrifuged (1,000 x g for 5 minutes), and the supernatant solution was removed. Each of the obtained pellets was suspended in 1 mL of PBS(-), respectively, and was divided into 900 μL for RNA purification by immunoprecipitation and 100 μL for Total RNA purification. After centrifugal separation (1,000 x g for 5 minutes), the supernatant solution was removed, to obtain miR-122 introduced cell pellet and GL3 introduced cell pellet for RNA purification by immunoprecipitation and for Total RNA purification.

[Preparation of anti-human AGO2 antibody immobilization solution]

To 50 μL of 1 mg/mL anti-human AGO2 antibody (produced by Wako Pure Chemical Industries Ltd.), 450 μL of buffer solution for protein immobilization (produced by Sumitomo Bakelite Co., Ltd.) was added and mixed, to prepare a solution for immobilizing 5 μg anti-human AGO2 antibody to bead.

[Preparation of anti-human AGO2 antibody-immobilized carrier solution]

To 10 mg of the particle for protein immobilization obtained in Experimental Example 1 (1), anti-human AGO2 antibody immobilization solution prepared as described above was added, and the mixture was mixed by inversion at 37°C for 4 hours. After centrifugal separation (3,000 x g for 1 minute), the supernatant solution was removed using pipette, and then the particle was washed twice with 500 μL of PBS (pH 7.4). After that, 500 μL of the buffer solution for inactivation (produced by Sumitomo Bakelite Company., Ltd.) was added and the mixture was mixed by inversion at room temperatures for 1 hour. After centrifugal separation (3,000 x g for 1 minute), the supernatant solution was removed using pipette, and then the particle was washed 5 times with 500 μL of PBS (pH 7.4), and suspended in 1 mL of PBS (pH 7.4). The resultant suspension was used as an anti-human AGO2 antibody-immobilized carrier solution.

[Purification of RNA by immunoprecipitation method]

To each of the miR-122 introduced cell pellet and the GL3 introduced cell pellet which were divided to be used for RNA purification by immunoprecipitation, 1 mL of cell lysis solution (0.05w/v% NP-40 (produced by Wako Pure Chemical Industries Ltd.) containing 20 mM Tris-HCl, 200 mM sodium chloride, and 2.5 mM magnesium chloride) was

added, and the cells where suspended by pipetting and allowed to stand on ice for 10 minutes. After centrifugal separation (20,000 x g for 20 minutes, at 4°C), the supernatant solution (cell lysate) was isolated.

After centrifugal separation (3,000 x g for 30 seconds) of 200 μL of the above-described anti-human AGO2 antibody-immobilized carrier solution (corresponding to 2 mg of beads), the supernatant solution was removed, then 1 mL each of the above-described cell lysate was added thereto, and the mixture was mixed by inversion in a refrigerator overnight. After centrifugal separation (3,000 x g for 30 seconds), the supernatant solution was removed, and the carrier was washed 3 times with 1 mL of the cell lysis solution. After that, 50 μL of 0.5w/v% SDS solution was added on the bead pellet, and the protein adsorbed on the carrier was eluted. To the eluate solution, 350 μL of sterilized water, and 400 μL of phenol/chloroform/isoamyl alcohol (25:24:1) were added and the solution was mixed by vortex mixer, then centrifuged (20,000 x g, 10 minutes). The upper layer was isolated, and 400 μL of chloroform was added thereto. The solution was mixed by vortex mixer, then centrifuged (20,000 x g, 10 minutes). The upper layer was isolated, and 3 μL of Ethachinmate (produced by Nippon Gene Co., Ltd.), 40 μL of 3 M sodium acetate, 1 mL of ethanol were added thereto. The solution was mixed by vortex mixer, then centrifuged (20,000 x g, 15 minutes). The precipitate was washed with 1 mL of 70v/v% ethanol, and air dried at room temperature for 20 minutes, then dissolved in 50 μL of sterilized water, to obtain a solution of immunoprecipitation purified RNA derived from miR-122 introduced cell and a solution of immunoprecipitation purified RNA derived from GL3 introduced cell.

[Purification of Total RNA]

To the miR-122 introduced cell pellet and the GL3 introduced cell pellet which were divided to be used for purifying Total RNA, 1 mL of ISOGEN (produced by Nippon Gene Co., Ltd.) was added, and the cells were lysed by pipetting, then 0.2 mL of chloroform was added and mixed with vortex mixer. After standing on ice for 1 hour, the mixture was subjected to centrifugal separation (20,000 x g, 10 minutes), and the upper layer was isolated. To the isolated solution, 0.6 mL of chloroform was added and mixed with vortex mixer, and the solution was centrifuged (20,000 x g, 10 minutes). The upper layer was isolated, and 0.6 mL of isopropanol was added, mixed, and centrifuged (20,000 x g, 10 minutes). The precipitate was washed with 1 mL of 70 v/v% ethanol, and air dried at room temperature for 20 minutes, then dissolved in 50 μL of sterilized water, to obtain a purified RNA solution derived from miR-122 introduced cell and a purified RNA solution derived from GL3 introduced cell.

[The reverse transcription reaction of miRNA and quantitative PCR]

A reverse transcription reaction of miRNA was carried out using TaqMan MicroRNA Reverse Transcription Kit (produced by Applied Biosystems Inc.) and TaqMan MicroRNA Assay Kit (for hsa-miR-122, for hsa-miR-21) as follows.

To 1 μL of 100 fold diluted solution which was prepared by adding 99 μL of purified water to 1 μL of the above-described immunoprecipitation purified RNA derived from miR-122 introduced cell, or to 1 μL of 100 fold diluted solution which was prepared by adding 99 μL of purified water to 1 μL of the above-described immunoprecipitation purified RNA derived from GL3 introduced cell, 0.15 μL of 100 mM dNTP mix, 0.75 μL of 10 x RT buffer solution, 0.19 μL of 20 U/μL RNase Inhibitor, 1 μL of 50 U/μL Multiscribe RT enzyme, 3 μL of 5 x RT primer (for hsa-miR-122 or has-miR-21), 8.16 μL of purified water were added respectively, to prepare 2 sorts of 15 μL of reaction solutions. After carrying out incubation at 16°C for 30 minutes, and at 42°C for 30 minutes, each reaction solution was further incubated at 85°C for 5 minutes, to obtain the reverse transcription reaction products.

To 1.33 μL of each reverse transcription reaction product, 10 μL of TaqMan 2 x PCR Master Mix (produced by Applied Biosystems Inc.), 1 μL of 20 x TaqMan Assay Mix (for hsa-miR-122 or hsa-miR-21), and 7.67 μL of purified water were added, respectively, to prepare total 20 μL of reaction solution, then using ABI7500 Fast Real-Time PCR System (produced by Applied Biosystems Inc.), quantitative PCR detection was carried out, and Ct values were determined, respectively. On the other hand, single-stranded synthetic miR-122 (5'-UGGAGUGUGACAAUGGUGUUUGU-3') or single-stranded synthetic miR-21 (5'-UAGCUUAUCAGACUGAUGUUGA-3') was used as a standard RNA, and diluted solutions by 10 fold dilution with purified water to $10^1$ to $10^5$ copies/μL, were prepared, and the reverse transcription reaction was carried out by the same procedure as described above, and quantitative PCR detection was performed. Standard curves of miR-122 and miR-21 were prepared from Ct values and its number of molecules of each of the obtained diluted standard RNA solution. Using said standard curve, numbers of molecules of miR-122 and miR-21 were calculated from each Ct value obtained as described above. Results of the quantitative determination for miR21 and miR-122 derived from miR-122 introduced cell as well as miR-21 and miR-122 derived from GL3 introduced cell are shown in Fig. 5, respectively.

From the results shown in Fig. 5, it can be confirmed that the miR-122 which is not observed in GL3 introduced cell is detected in the miR-122 introduced cell, and the miR-122 has been evidently introduced into HepG2 cell.

[Reverse transcription reaction and quantitative PCR of mRNA]

Reverse transcription reaction of mRNA was performed using SuperSript VILO cDNA Synthesis Kit (produced by Invitrogen Corporation) as follows.

That is, to 14 μL each of the immunoprecipitation purified RNA solution derived from the above-described miR-122-introduced cell or the immunoprecipitation purified RNA solution derived from GL3 cell, 4 μL of 5 x VILO Reaction Mix and 2 μL of 10 x SuperScript Mix were added respectively and mixed, to prepare 2 sorts of and total 20 μL each of immunoprecipitation purified RNA reaction solutions. In addition, to 2 μL each of the purified Total RNA derived from the above-described miR-122-introduced cell and the purified Total RNA derived from GL3 cell, 4 μL of 5 x VILO Reaction Mix, 2 μL of 10 x SuperScript Mix, and 12 μL of purified water were added respectively and mixed, to prepare 2 sorts of and total 20 μL each of Total RNA reaction solutions. The immunoprecipitation purified RNA reaction solution and the Total RNA reaction solution were each incubated at 25°C for 10 minutes, then at 42°C for 60 minutes, and followed by incubation at 85°C for 5 minutes, to obtain reverse transcription reaction products.

On the other hand, as a PCR primer for human aldolase A (AldoA) cDNA (GenBank Accession: NM_000034) amplification, a Forward primer (GTTGTGGGCATCAAGGT) and a Reverse primer (CAATCTTCAGCACACAACG) were synthesized; as a PCR primer for human cathionic amino acid transporter-1 (CAT-1) cDNA (GenBank Accession: NM_003045) amplification, a Forward primer (GTTCCAGAGGGAGCATC) and a Reverse primer (CAAGCAAATAACTACCAGGTC) were synthesized; and as a PCR primer for human GAPDH cDNA (GenBank Accession: NM_002046) amplification, a Forward primer (AATCCCATCACCATCTTCC) and a Reverse primer (GCAGAGATGATGACCCTTT) were synthesized, respectively.

To 1 μL each of the reverse transcription reaction products of the immunoprecipitation purified RNA reaction solution obtained as described above, 0.8 μL of 5 μM Forward primer and 0.8 μL of 5 μM Reverse primer for respective cDNA synthesis, 10 μL of 2 x Power SYBR Master Mix (produced by Applied Biosystems Inc.), 7.4 μL of purified water were added respectively, to prepare total 20 μL of each reaction solution, and quantitative PCR detection of cach reaction solution was performed using AB17500 Fast Real-Time PCR System (produced by Applied Biosystems Inc.), to obtain Ct value of AldoA, Ct value of CAT-1 and Ct value of GAPDH which were derived from miR-122 introduced cell, and Ct value of AldoA, Ct value of CAT-1 and Ct value of GAPDH which were derived from GL3 introduced cell. In addition, using each reverse transcription reaction product of the Total RNA reaction solution obtained as described above, quantitative PCR detection was carried out by the same method as used for the reverse transcription reaction product of the above-described immunoprecipitation purified RNA reaction solution, to obtain Ct value of AldoA, Ct value of CAT-1 and Ct value of GAPDH which were derived from miR-122 introduced cell, and Ct value of AldoA, Ct value of CAT-1 and Ct value of GAPDH which were derived from GL3 introduced cell.

As to each AldoA, CAT-1 and GAPDH of the immunoprecipitation purified RNA, and as to each AldoA, CAT-1 and GAPDH of the total RNA, the value after subtraction of the Ct value of RNA derived from GL3 introduced cell from the Ct value of RNA derived from miR-122 introduced cell was calculated by the following equation:

$$\log_2 [-(\text{Ct value of the RNA derived from GL3 cell} - \text{Ct value of the RNA derived from miR-122 cell})]$$

and increase or decrease of each amount of mRNA of miR-122 introduced cell to GL3 introduced cell was compared. Results are shown in Fig. 6.

According to Joacim et. al., Nucleic Acid Research, 36(4), 2008, it has been reported that Aldorase A (AldoA) has a seed sequence which serves as a target of miR-122 in the 3'UTR region of mRNA, that is, a target mRNA of miR-122. In addition, according to Suvendra et.al., Cell, 125, and 1111-1124, 2006, it has been reported that cationic amino acid transporter1 (CAT-1) is also a target mRNA of miR-122. On the other hand, since firefly luciferase siRNA (GL3) is not miRNA, even if it is introduce into HepG2, it does not bind to mRNA such as AldoA and CAT-1. Therefore, the GL3 introduced cell in this experiment serves as a control (standard) of a miR-122 introduced cell.

As shown in Fig. 6, as to AldoA and CAT-1 mRNA which are a target of miR-122, although amount of the total mRNA in the miR-122 introduced cell decreased as compared with GL3 introduced cell, amount of mRNA in the Ago2 immunoprecipitated RNA was increased. On the other hand, in the GAPDH mRNA which has no target sequence of miR-122, there was no significant difference in Ct value between total RNA and Ago2 immunoprecipitated RNA. From the facts mentioned above, it can be found out that by performing Ago2 immunoprecipitation method of the present invention using miR-122 introduced HepG2 cell, AldoA and CAT-1 which are the target mRNA of miR-122 can be obtained efficiently. Namely, from this results, it is demonstrated that, by expressing specified microRNA excessively, the microRNA and target mRNA thereof are concentrated specifically in the Ago2 immunoprecipitated RNA, and further it can be clarified that, by employing the method of the present invention, target nucleic acid of small RNA such as a target mRNA of microRNA can be obtained efficiently, and the analysis of the target nucleic acid of mRNA can be performed efficiently

as well.

## Claims

1. A carrier for obtaining a small RNA produced by introducing a polymerizable functional group or a chain transfer group to the surface of a core particle, mixing said particle with a polymerizable monomer, then promoting polymerization reaction to form a layer comprising a polymer compound on the surface of said particle, and immobilizing a physiologically active substance having affinity to a small RNA-binding protein on the surface of the particle.

2. The carrier according to claim 1, wherein the physiologically active substance is an antibody.

3. The carrier according to claim 2, wherein the antibody is an anti-Argonaute-family protein antibody.

4. The carrier according to claim 1, wherein the above-described polymerizable monomer is the one which comprises an ethylenically unsaturated polymerizable monomer (a) having at least a functional group which can immobilize a physiologically active substance, or the one which further comprises an ethylenically unsaturated polymerizable monomer (b) having an alkylene oxy group.

5. The carrier according to claim 4, wherein

   (i) the functional group of the ethylenically unsaturated polymerizable monomer (a) having a functional group which can immobilize a physiologically active substance is at least one functional group selected from aldehyde group, active ester group, epoxy group, vinyl sulfone group, and biotin; or
   (ii) the ethylenically unsaturated polymerizable monomer (a) having a functional group which can immobilize a physiologically active substance is a monomer having an active ester group represented by the following general formula [1]

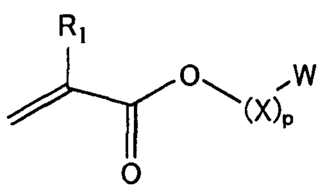

[1]

(in the above formula, $R_1$ represents a hydrogen atom or a methyl group; X represents an alkylene oxy group or an alkylene group having 1 to 10 carbon atoms; W represents an active ester group or a p-nitrophenyl active ester group; p represents an integer of 1 to 100; when p is 2 to 100, each repeating unit X may be either identical or different); or

(iii) the ethylenically unsaturated polymerizable monomer (b) having an alkylene oxy group is a monomer represented by the following general formula [2]

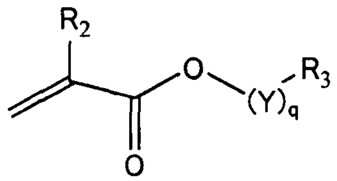

[2]

(in the above formula, $R_2$ represents a hydrogen atom or a methyl group; $R_3$ represents a hydrogen atom or an alkyl group having 1 to 20 carbon atoms; Y represents an alkylene oxy group having 1 to 10 carbon atoms; q represents an integer of 1 to 100; each repeating unit Y may be either identical or different); or

(iv) the ethylenically unsaturated polymerizable monomer (b) having an alkylene oxy group is methoxypolyethylene glycol (meth)acrylate; or

(v) the ethylenically unsaturated polymerizable monomer (a) having a functional group which can immobilize a physiologically active substance is p-nitrophenyloxycarbonyl-polyethylene glycol (meth)acrylate, and the ethylenically unsaturated polymerizable monomer (b) having an alkylene oxy group is methoxypolyethylene glycol (meth)acrylate.

**6.** Use of the carriers according to any one of claims 1 to 5 in a method for obtaining a small RNA.

**7.** A method for obtaining a small RNA by using the carrier according to any one of claims 1 to 5, wherein the method comprises the following steps:

(1) introducing a polymerizable functional group or a chain transfer group to the surface of core particle, mixing said particle with a polymerizable monomer, subsequently promoting polymerization reaction to form a layer comprising a polymer compound on the surface of said particle, and immobilizing a physiologically active substance having affinity to small RNA-binding protein on the surface of said particle to form a carrier, and contacting the carrier with a complex of said small RNA-binding protein and a small RNA (small RNA-binding protein-small RNA complex) to form a bound substance of said physiologically active substance and said small RNA-binding protein-small RNA complex on the surface of said carrier;

(2) isolating the obtained carrier having a bound substance of physiologically active substance and small RNA-binding protein-small RNA complex on the surface thereof; and

(3) eluting the small RNA from said bound substance of physiologically active substance and small RNA-binding protein-small RNA complex.

**8.** The method for obtaining a small RNA according to claim 7, wherein the physiologically active substance is an antibody.

**9.** The method of claim 8, wherein the antibody is an anti-Argonaute-family protein antibody.

**10.** The method for obtaining a small RNA according to any one of claims 7 to 9, wherein the above-described polymerizable monomer is the one which comprises an ethylenically unsaturated polymerizable monomer (a) having at least a functional group which can immobilize a physiologically active substance, or the one which further comprises an ethylenically unsaturated polymerizable monomer (b) having an alkylene oxy group.

**11.** The method for obtaining a small RNA according to claim 10, wherein

(i) the functional group of the ethylenically unsaturated polymerizable monomer (a) having a functional group which can immobilize a physiologically active substance is at least one functional group selected from aldehyde group, active ester group, epoxy group, vinyl sulfone group, and biotin; or

(ii) the ethylenically unsaturated polymerizable monomer (a) having a functional group which can immobilize a

physiologically active substance is a monomer having an active ester group represented by the following general formula [1]

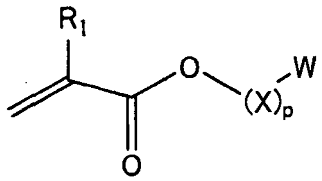

[1]

(in the above formula, $R_1$ represents a hydrogen atom or a methyl group; X represents an alkylene oxy group or an alkylene group having 1 to 10 carbon atoms; W represents an active ester group or a p-nitrophenyl active ester group; p represents an integer of 1 to 100; when p is 2 to 100, each repeating unit X may be either identical or different); or

(iii) the ethylenically unsaturated polymerizable monomer (b) having an alkylene oxy group is a monomer represented by the following general formula [2]

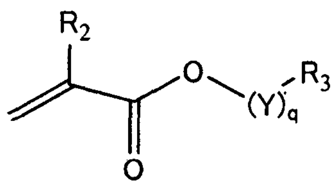

[2]

(in the above formula, $R_2$ represents a hydrogen atom or a methyl group; $R_3$ represents a hydrogen atom or an alkyl group having 1 to 20 carbon atoms; Y represents an alkylene oxy group having 1 to 10 carbon atoms; q represents an integer of 1 to 100; each repeating unit Y may be either identical or different); or

(iv) the ethylenically unsaturated polymerizable monomer (b) having an alkylene oxy group is methoxypolyethylene glycol (meth)acrylate; or

(v) the ethylenically unsaturated polymerizable monomer (a) having a functional group which can immobilize a physiologically active substance is p-nitrophenyloxycarbonyl-polyethylene glycol (metha)acrylate, and the ethylenically unsaturated polymerizable monomer (b) having an alkylene oxy group is methoxypolyethylene glycol (meth)acrylate.

**12.** A reagent for obtaining a small RNA and/or a target nucleic acid of small RNA, comprising the carrier according to claim 1.

**13.** A reagent kit for obtaining a small RNA and/or a target nucleic acid of small RNA, comprising the reagent according to claim 12.

**14.** Use of the carrier of claim 1 in a method for obtaining a target nucleic acid of small RNA.

**15.** A method for obtaining a target nucleic acid of small RNA by using the carrier according to claim 1, wherein the method comprises the following steps:

(1) introducing a polymerizable functional group or a chain transfer group to the surface of core particle, mixing said particle with a polymerizable monomer, subsequently promoting polymerization reaction to form a layer comprising a polymer compound on the surface of said particle, and immobilizing a physiologically active substance having affinity to small RNA-binding protein on the surface of said particle to form a carrier, and contacting the carrier with a complex of said small RNA-binding protein,small RNA and a target nucleic acid of small RNA (small RNA-binding protein-small RNA-target nucleic acid of small RNA complex) to form a bound substance of said physiologically active substance and said small RNA-binding protein-small RNA-target nucleic acid of small RNA complex on the surface of said carrier;

(2) isolating the obtained carrier having the bound substance of physiologically active substance and small RNA-binding protein-small RNA -target nucleic acid of small RNA complex on the surface thereof; and

(3) eluting the bound substance of small RNA-target nucleic acid of small RNA from said bound substance of

physiologically active substance and small RNA-binding protein-small RNA - target nucleic acid of small RNA complex.

## Patentansprüche

1. Träger zur Gewinnung einer kleinen RNA, erzeugt durch Einführen einer polymerisierbaren funktionellen Gruppe oder einer Kettenübertragungsgruppe auf die Oberfläche eines Kernteilchens, Mischen des Teilchens mit einem polymerisierbaren Monomer, dann Fördern einer Polymerisationsreaktion unter Bildung einer Schicht, die eine Polymerverbindung umfasst, auf der Oberfläche des Teilchens und Immobilisieren einer physiologisch aktiven Substanz, die Affinität zu einem kleine RNA bindenden Protein aufweist, auf der Oberfläche des Teilchens.

2. Träger gemäß Anspruch 1, wobei die physiologisch aktive Substanz ein Antikörper ist.

3. Träger gemäß Anspruch 2, wobei der Antikörper ein Anti-Argonautenprotein-Antikörper ist.

4. Träger gemäß Anspruch 1, wobei das oben beschriebene polymerisierbare Monomer ein ethylenisch ungesättigtes polymerisierbares Monomer (a) umfasst, das wenigstens eine funktionelle Gruppe, die eine physiologisch aktive Substanz immobilisieren kann, aufweist, oder weiterhin ein ethylenisch ungesättigtes polymerisierbares Monomer (b) umfasst, das eine Alkylenoxy-Gruppe aufweist.

5. Träger gemäß Anspruch 4, wobei

(i) die funktionelle Gruppe des ethylenisch ungesättigten polymerisierbaren Monomers (a), das eine funktionelle Gruppe, die eine physiologisch aktive Substanz immobilisieren kann, aufweist, wenigstens eine funktionelle Gruppe ist, die aus einer Aldehydgruppe, aktiven Estergruppe, Epoxygruppe, Vinylsulfongruppe und Biotin ausgewählt ist; oder
(ii) das ethylenisch ungesättigte polymerisierbare Monomer (a), das eine funktionelle Gruppe, die eine physiologisch aktive Substanz immobilisieren kann, aufweist, ein Monomer ist, das eine aktive Estergruppe aufweist und durch die folgende allgemeine Formel [1] dargestellt wird:

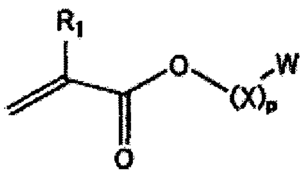

[1]

(in der obigen Formel steht $R_1$ für ein Wasserstoffatom oder eine Methylgruppe; X steht für eine Alkylenoxygruppe oder eine Alkylengruppe mit 1 bis 10 Kohlenstoffatomen; W steht für eine aktive Estergruppe oder eine p-Nitrophenyl-aktive-Estergruppe; p steht für eine ganze Zahl von 1 bis 100; wenn p 2 bis 100 ist, können die Repetiereinheiten X jeweils gleich oder verschieden sein); oder
(iii) das ethylenisch ungesättigte polymerisierbare Monomer (b), das eine Alkylenoxygruppe aufweist, ein Monomer ist, das durch die folgende allgemeine Formel [2] dargestellt wird:

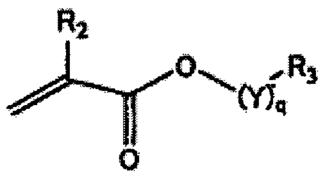

[2]

(in der obigen Formel steht $R_2$ für ein Wasserstoffatom oder eine Methylgruppe; $R_3$ steht für ein Wasserstoffatom

oder eine Alkylgruppe mit 1 bis 20 Kohlenstoffatomen; Y steht für eine Alkylenoxygruppe mit 1 bis 10 Kohlenstoffatomen; q steht für eine ganze Zahl von 1 bis 100; die Repetiereinheiten Y können jeweils gleich oder verschieden sein); oder

(iv) es sich bei dem ethylenisch ungesättigten polymerisierbaren Monomer (b), das eine Alkylenoxygruppe aufweist, um Methoxypolyethylenglycol-(meth)acrylat handelt; oder

(v) es sich bei dem ethylenisch ungesättigten polymerisierbaren Monomer (a), das eine funktionelle Gruppe, die eine physiologisch aktive Substanz immobilisieren kann, aufweist, um p-Nitrophenyloxycarbonylpolyethylenglycol(meth)acrylat handelt und es sich bei dem ethylenisch ungesättigten polymerisierbaren Monomer (b), das eine Alkylenoxygruppe aufweist, um Methoxypolyethylenglycol(meth)acrylat handelt.

**6.** Verwendung der Träger gemäß einem der Ansprüche 1 bis 5 in einem Verfahren zur Gewinnung einer kleinen RNA.

**7.** Verfahren zur Gewinnung einer kleinen RNA unter Verwendung des Trägers gemäß einem der Ansprüche 1 bis 5, wobei das Verfahren die folgenden Schritte umfasst:

(1) Einführen einer polymerisierbaren funktionellen Gruppe oder einer Kettenübertragungsgruppe auf die Oberfläche eines Kernteilchens, Mischen des Teilchens mit einem polymerisierbaren Monomer, anschließend Fördern einer Polymerisationsreaktion unter Bildung einer Schicht, die eine Polymerverbindung umfasst, auf der Oberfläche des Teilchens und Immobilisieren einer physiologisch aktiven Substanz, die Affinität zu einem kleine RNA bindenden Protein aufweist, auf der Oberfläche des Teilchens unter Bildung eines Trägers und In-Kontakt-Bringen des Trägers mit einem Komplex aus dem kleine RNA bindenden Protein und einer kleinen RNA (kleine-RNA-bindendes-Protein-kleine-RNA-Komplex) unter Bildung einer gebundenen Substanz aus der physiologisch aktiven Substanz und dem kleine-RNA-bindendes-Protein-kleine-RNA-Komplex auf der Oberfläche des Trägers;

(2) Isolieren des erhaltenen Trägers, der eine gebundene Substanz aus der physiologisch aktiven Substanz und dem kleine-RNA-bindendes-Protein-kleine-RNA-Komplex auf seiner Oberfläche aufweist; und

(3) Eluieren der kleinen RNA ausgehend von der gebundenen Substanz aus der physiologisch aktiven Substanz und dem kleine-RNA-bindendes-Protein-kleine-RNA-Komplex.

**8.** Verfahren zur Gewinnung einer kleinen RNA gemäß Anspruch 7, wobei die physiologisch aktive Substanz ein Antikörper ist.

**9.** Verfahren gemäß Anspruch 8, wobei der Antikörper ein Anti-Argonautenprotein-Antikörper ist.

**10.** Verfahren zur Gewinnung einer kleinen RNA gemäß einem der Ansprüche 7 bis 9, wobei das oben beschriebene polymerisierbare Monomer ein ethylenisch ungesättigtes polymerisierbares Monomer (a) umfasst, das wenigstens eine funktionelle Gruppe, die eine physiologisch aktive Substanz immobilisieren kann, aufweist, oder weiterhin ein ethylenisch ungesättigtes polymerisierbares Monomer (b) umfasst, das eine AlkylenoxyGruppe aufweist.

**11.** Verfahren zur Gewinnung einer kleinen RNA gemäß Anspruch 10, wobei

(i) die funktionelle Gruppe des ethylenisch ungesättigten polymerisierbaren Monomers (a), das eine funktionelle Gruppe, die eine physiologisch aktive Substanz immobilisieren kann, aufweist, wenigstens eine funktionelle Gruppe ist, die aus einer Aldehydgruppe, aktiven Estergruppe, Epoxygruppe, Vinylsulfongruppe und Biotin ausgewählt ist; oder

(ii) das ethylenisch ungesättigte polymerisierbare Monomer (a), das eine funktionelle Gruppe, die eine physiologisch aktive Substanz immobilisieren kann, aufweist, ein Monomer ist, das eine aktive Estergruppe aufweist und durch die folgende allgemeine Formel [1] dargestellt wird:

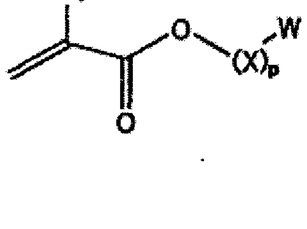

[1]

(in der obigen Formel steht $R_1$ für ein Wasserstoffatom oder eine Methylgruppe; X steht für eine Alkylenoxygruppe oder eine Alkylengruppe mit 1 bis 10 Kohlenstoffatomen; W steht für eine aktive Estergruppe oder eine p-Nitrophenyl-aktive-Estergruppe; p steht für eine ganze Zahl von 1 bis 100; wenn p 2 bis 100 ist, können die Repetiereinheiten X jeweils gleich oder verschieden sein); oder

(iii) das ethylenisch ungesättigte polymerisierbare Monomer (b), das eine Alkylenoxygruppe aufweist, ein Monomer ist, das durch die folgende allgemeine Formel [2] dargestellt wird:

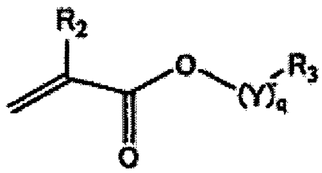

[2]

(in der obigen Formel steht $R_2$ für ein Wasserstoffatom oder eine Methylgruppe; $R_3$ steht für ein Wasserstoffatom oder eine Alkylgruppe mit 1 bis 20 Kohlenstoffatomen; Y steht für eine Alkylenoxygruppe mit 1 bis 10 Kohlenstoffatomen; q steht für eine ganze Zahl von 1 bis 100; die Repetiereinheiten Y können jeweils gleich oder verschieden sein); oder

(iv) es sich bei dem ethylenisch ungesättigten polymerisierbaren Monomer (b), das eine Alkylenoxygruppe aufweist, um Methoxypolyethylenglycol-(meth)acrylat handelt; oder

(v) es sich bei dem ethylenisch ungesättigten polymerisierbaren Monomer (a), das eine funktionelle Gruppe, die eine physiologisch aktive Substanz immobilisieren kann, aufweist, um p-Nitrophenyloxycarbonylpolyethylenglycol(meth)acrylat handelt und es sich bei dem ethylenisch ungesättigten polymerisierbaren Monomer (b), das eine Alkylenoxygruppe aufweist, um Methoxypolyethylenglycol(meth)acrylat handelt.

**12.** Reagens zur Gewinnung einer kleinen RNA und/oder einer Zielnucleinsäure von kleiner RNA, umfassend den Träger gemäß Anspruch 1.

**13.** Reagens-Kit zur Gewinnung einer kleinen RNA und/oder einer Zielnucleinsäure von kleiner RNA, umfassend das Reagens gemäß Anspruch 12.

**14.** Verwendung des Trägers gemäß Anspruch 1 in einem Verfahren zur Gewinnung einer Zielnucleinsäure von kleiner RNA.

**15.** Verfahren zur Gewinnung einer Zielnucleinsäure von kleiner RNA unter Verwendung des Trägers gemäß Anspruch 1, wobei das Verfahren die folgenden Schritte umfasst:

(1) Einführen einer polymerisierbaren funktionellen Gruppe oder einer Kettenübertragungsgruppe auf die Oberfläche eines Kernteilchens, Mischen des Teilchens mit einem polymerisierbaren Monomer, anschließend Fördern einer Polymerisationsreaktion unter Bildung einer Schicht, die eine Polymerverbindung umfasst, auf der Oberfläche des Teilchens und Immobilisieren einer physiologisch aktiven Substanz, die Affinität zu einem kleine RNA bindenden Protein aufweist, auf der Oberfläche des Teilchens unter Bildung eines Trägers und In-Kontakt-Bringen des Trägers mit einem Komplex aus dem kleine RNA bindenden Protein, einer kleinen RNA und einer Zielnucleinsäure der kleinen RNA (kleine-RNA-bindendes-Protein-kleine-RNA-Zielnucleinsäure-der-kleinen-RNA-Komplex) unter Bildung einer gebundenen Substanz aus der physiologisch aktiven Substanz und dem kleine-RNA-bindendes-Protein-kleine-RNA-Zielnucleinsäure-derkleinen-RNA-Komplex auf der Oberfläche des Trägers;

(2) Isolieren des erhaltenen Trägers, der eine gebundene Substanz aus der physiologisch aktiven Substanz und dem kleine-RNA-bindendes-Protein-kleine-RNA-Zielnucleinsäure-der-kleinen-RNA-Komplex auf seiner Oberfläche aufweist; und

(3) Eluieren der gebundenen Substanz aus der kleinen RNA und der Zielnucleinsäure der kleinen RNA ausgehend von der gebundenen Substanz aus der physiologisch aktiven Substanz und dem kleine-RNA-bindendes-Protein-kleine-RNA-Zielnucleinsäure-der-kleinen-RNA-Komplex.

**Revendications**

1. Support pour obtenir un petit ARN produit par introduction d'un groupe fonctionnel polymérisable ou d'un groupe de transfert de chaîne sur la surface d'une particule centrale, mélange de ladite particule à un monomère polymérisable, puis promotion d'une réaction de polymérisation pour former une couche comprenant un composé polymère sur la surface de ladite particule, et immobilisation, sur la surface de la particule, d'une substance physiologiquement active ayant une affinité pour une protéine de liaison à un petit ARN.

2. Support selon la revendication 1, dans lequel la substance physiologiquement active est un anticorps.

3. Support selon la revendication 2, dans lequel l'anticorps est un anticorps dirigé contre les protéines de la famille des Argonautes.

4. Support selon la revendication 1, dans lequel le monomère polymérisable décrit ci-dessus est un monomère qui comprend un monomère polymérisable à insaturation éthylénique (a) ayant au moins un groupe fonctionnel qui peut immobiliser une substance physiologiquement active, ou un monomère qui comprend en outre un monomère polymérisable à insaturation éthylénique (b) ayant un groupe alkylène-oxy.

5. Support selon la revendication 4, dans lequel

(i) le groupe fonctionnel du monomère polymérisable à insaturation éthylénique (a) ayant un groupe fonctionnel qui peut immobiliser une substance physiologiquement active est au moins un groupe fonctionnel choisi parmi un groupe aldéhyde, un groupe ester actif, un groupe époxy, un groupe vinylsulfone et la biotine ; ou
(ii) le monomère polymérisable à insaturation éthylénique (a) ayant un groupe fonctionnel qui peut immobiliser une substance physiologiquement active est un monomère ayant un groupe ester actif représenté par la formule générale (1) ci-après

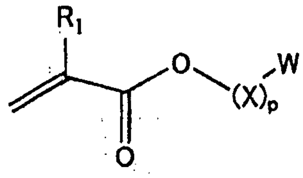

[1]

(dans la formule ci-dessus, $R_1$ représente un atome d'hydrogène ou un groupe méthyle ; X représente un groupe alkylène-oxy ou un groupe alkylène ayant 1 à 10 atomes de carbone ; W représente un groupe ester actif ou un groupe ester actif p-nitrophényle ; p représente un entier de 1 à 100 ; quand p vaut 2 à 100, chaque unité répétitive X peut être identique ou différente) ; ou
(iii) le monomère polymérisable à insaturation éthylénique (b) ayant un groupe alkylène-oxy est un monomère représenté par la formule générale (2)

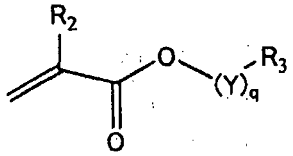

[2]

(dans la formule ci-dessus, $R_2$ représente un atome d'hydrogène ou un groupe méthyle ; $R_3$ représente un atome d'hydrogène ou un groupe alkyle ayant 1 à 20 atomes de carbone ; Y représente un groupe alkylène-oxy ayant 1 à 10 atomes de carbone ; q représente un entier de 1 à 100 ; chaque unité répétitive Y peut être identique ou différente) ; ou
(iv) le monomère polymérisable à insaturation éthylénique (b) ayant un groupe alkylène-oxy est le (méth)acrylate de méthoxypolyéthylèneglycol ; ou
(v) le monomère polymérisable à insaturation éthylénique (a) ayant un groupe fonctionnel qui peut immobiliser

une substance physiologiquement active est le (méth)acrylate de p-nitrophényloxycarbonyl-polyéthylèneglycol, et le monomère polymérisable à insaturation éthylénique (b) ayant un groupe alkylène-oxy est le (méth)acrylate de méthoxypolyéthylèneglycol.

6. Utilisation des supports selon l'une quelconque des revendications 1 à 5 dans un procédé d'obtention d'un petit ARN.

7. Procédé pour obtenir un petit ARN par utilisation du support selon l'une quelconque des revendications 1 à 5, le procédé comprenant les étapes suivantes :

(1) introduction d'un groupe fonctionnel polymérisable ou d'un groupe de transfert de chaîne sur la surface d'une particule centrale, mélange de ladite particule à un monomère polymérisable, puis promotion d'une réaction de polymérisation pour former une couche comprenant un composé polymère sur la surface de ladite particule, et immobilisation d'une substance physiologiquement active ayant une affinité pour une protéine de liaison à un petit ARN sur la surface de ladite particule pour former un support, et mise en contact du support avec un complexe de ladite protéine de liaison à un petit ARN et d'un petit ARN (complexe protéine de liaison à un petit ARN-petit ARN) pour former une substance liée de ladite substance physiologiquement active et dudit complexe protéine de liaison à un petit ARN-petit ARN sur la surface dudit support ;
(2) isolement du support obtenu ayant sur sa surface une substance liée de la substance physiologiquement active et du complexe protéine de liaison à un petit ARN-petit ARN ; et
(3) élution du petit ARN à partir de ladite substance liée de la substance physiologiquement active et du complexe protéine de liaison à un petit ARN-petit ARN.

8. Procédé pour obtenir un petit ARN selon la revendication 7, dans lequel la substance physiologiquement active est un anticorps.

9. Procédé selon la revendication 8, dans lequel l'anticorps est un anticorps dirigé contre une protéine de la famille des Argonautes.

10. Procédé pour obtenir un petit ARN selon l'une quelconque des revendications 7 à 9, dans lequel le monomère polymérisable décrit ci-dessus est un monomère qui comprend un monomère polymérisable à insaturation éthylénique (a) ayant au moins un groupe fonctionnel qui peut immobiliser une substance physiologiquement active, ou un monomère qui comprend en outre un monomère polymérisable à insaturation éthylénique (b) ayant un groupe alkylène-oxy.

11. Procédé pour obtenir un petit ARN selon la revendication 10, dans lequel

(i) le groupe fonctionnel du monomère polymérisable à insaturation éthylénique (a) ayant un groupe fonctionnel qui peut immobiliser une substance physiologiquement active est au moins un groupe fonctionnel choisi parmi un groupe aldéhyde, un groupe ester actif, un groupe époxy, un groupe vinylsulfone et la biotine ; ou
(ii) le monomère polymérisable à insaturation éthylénique (a) ayant un groupe fonctionnel qui peut immobiliser une substance physiologiquement active est un monomère ayant un groupe ester actif représenté par la formule générale (1) ci-après

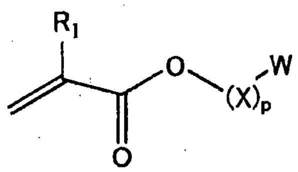
[1]

(dans la formule ci-dessus, $R_1$ représente un atome d'hydrogène ou un groupe méthyle ; X représente un groupe alkylène-oxy ou un groupe alkylène ayant 1 à 10 atomes de carbone ; W représente un groupe ester actif ou un groupe ester actif p-nitrophényle ; p représente un entier de 1 à 100 ; quand p vaut 2 à 100, chaque unité répétitive X peut être identique ou différente) ; ou
(iii) le monomère polymérisable à insaturation éthylénique (b) ayant un groupe alkylène-oxy est un monomère représenté par la formule générale (2)

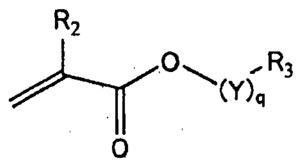

[2]

(dans la formule ci-dessus, $R_2$ représente un atome d'hydrogène ou un groupe méthyle ; $R_3$ représente un atome d'hydrogène ou un groupe alkyle ayant 1 à 20 atomes de carbone ; Y représente un groupe alkylène-oxy ayant 1 à 10 atomes de carbone ; q représente un entier de 1 à 100 ; chaque unité répétitive Y peut être identique ou différente) ; ou

(iv) le monomère polymérisable à insaturation éthylénique (b) ayant un groupe alkylène-oxy est le (méth)acrylate de méthoxypolyéthylèneglycol ; ou

(v) le monomère polymérisable à insaturation éthylénique (a) ayant un groupe fonctionnel qui peut immobiliser une substance physiologiquement active est le (méth)acrylate de p-nitrophényloxycarbonyl-polyéthylèneglycol, et le monomère polymérisable à insaturation éthylénique (b) ayant un groupe alkylène-oxy est le (méth)acrylate de méthoxypolyéthylèneglycol.

**12.** Réactif pour obtenir un petit ARN et/ou un acide nucléique cible d'un petit ARN, comprenant le support selon la revendication 1.

**13.** Trousse de réactifs pour obtenir un petit ARN et/ou un acide nucléique cible d'un petit ARN, comprenant le réactif selon la revendication 12.

**14.** Utilisation du support selon la revendication 1 dans un procédé pour obtenir un acide nucléique cible d'un petit ARN.

**15.** Procédé pour obtenir un acide nucléique cible d'un petit ARN par utilisation du support selon la revendication 1, le procédé comprenant les étapes suivantes :

(1) introduction d'un groupe fonctionnel polymérisable ou d'un groupe de transfert de chaîne sur la surface de la particule centrale, mélange de ladite particule à un monomère polymérisable, puis promotion- d'une réaction de polymérisation pour former une couche comprenant un composé polymère sur la surface de ladite particule, et immobilisation d'une substance physiologiquement active ayant une affinité pour une protéine de liaison à un petit ARN sur la surface de ladite particule pour former un support, et mise en contact du support avec un complexe de ladite protéine de liaison à un petit ARN, du petit ARN et d'un acide nucléique cible d'un petit ARN (complexe protéine de liaison à un petit ARN - petit ARN - acide nucléique cible d'un petit ARN) pour former un substance liée de ladite substance physiologiquement active et dudit complexe protéine de liaison à un petit ARN - petit ARN - acide nucléique cible d'un petit ARN, sur la surface dudit support ;

(2) isolement du support obtenu, ayant sur sa surface la substance liée de la substance physiologiquement active et du complexe protéine de liaison à un petit ARN - petit ARN - acide nucléique cible d'un petit ARN ; et

(3) élution de la substance liée du petit ARN - acide nucléique cible d'un petit ARN, à partir de ladite substance liée de la substance physiologiquement active et du complexe protéine de liaison à un petit ARN - petit ARN - acide nucléique cible d'un petit ARN.

Fig. 1

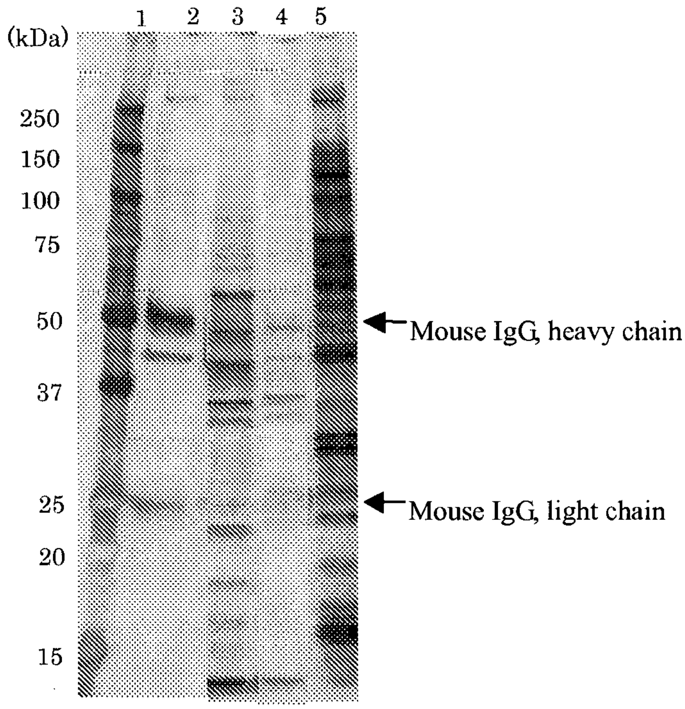


Lane 1: Molecular weight marker
Lane 2: Carrier employing the particle involved in the present invention
Lane 3: Carrier produced by Pierce
Lane 4: Carrier produced by GE
Lane 5: Carrier produced by DYNAL

Fig. 2

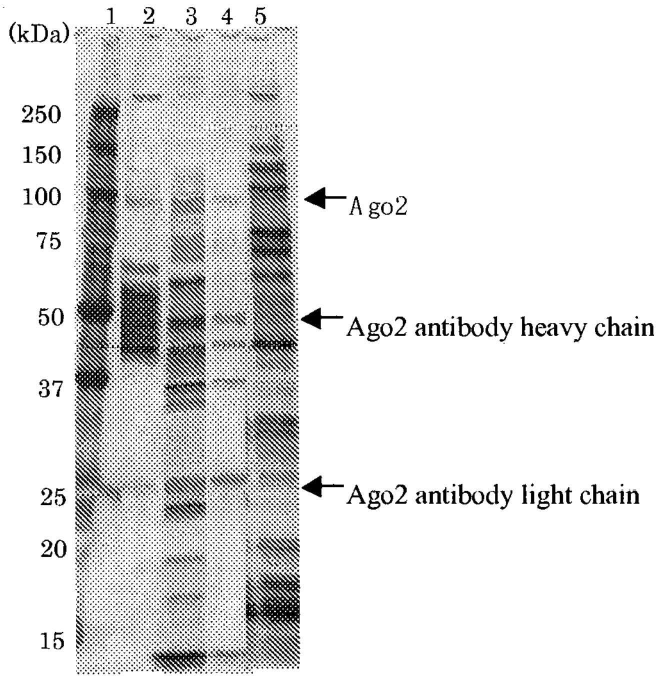


Lane 1: Molecular weight marker
Lane 2: Anti-Ago2 antibody-immobilized carrier employing the particle involved in the present invention
Lane 3: Anti-Ago2 antibody-immobilized carrier produced by Pierce
Lane 4: Anti-Ago2 antibody-immobilized carrier produced by GE
Lane 5: Anti-Ago2 antibody-immobilized carrier produced by DYNAL

Fig. 3

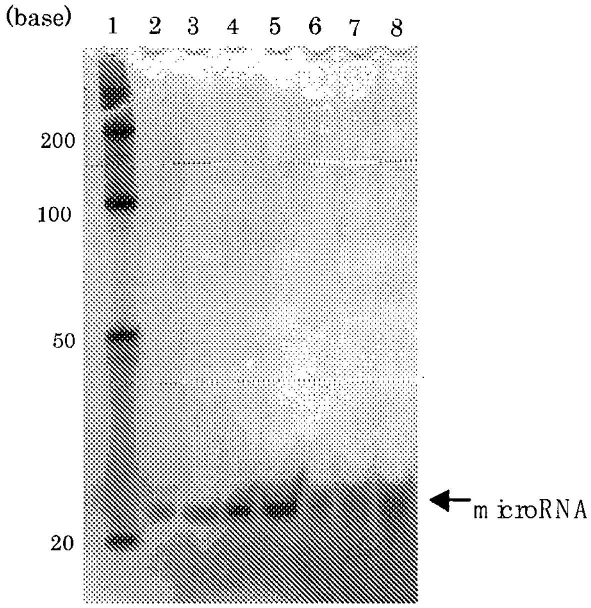


| |
|---|
| Lane 1: Molecular weight marker |
| Lane 2: Synthetic RNA Oligo (22nt), 0.25 ng |
| Lane 3: Synthetic RNA Oligo (22nt), 0.5 ng |
| Lane 4: Synthetic RNA Oligo (22nt), 1 ng |
| Lane 5: Synthetic RNA Oligo (22nt), 2 ng |
| Lane 6: RNA obtained by Anti-human Ago2 antibody 5 μg/2 mg carrier |
| Lane 7: RNA obtained by Anti-human Ago2 antibody 10 μg/2 mg carrier |
| Lane 8: RNA obtained by Anti-human Ago2 antibody 20 μg/2 mg carrier |

Fig. 4

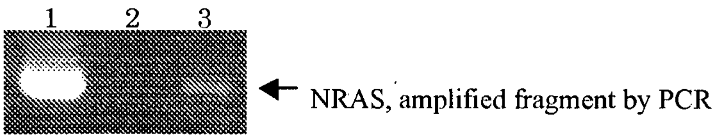


| |
|---|
| Lane 1: Template cDNA = derived from 1 μg TotalRNA |
| Lane 2: Template cDNA = derived from immunoprecipitation RNA (1 x 107 cell) using mouse IgG-immobilized carrier |
| Lane 3: Template cDNA = derived from immunoprecipitation RNA (1 x 107 cell) using anti-human Ago2-immobilized carrier |

Fig. 5

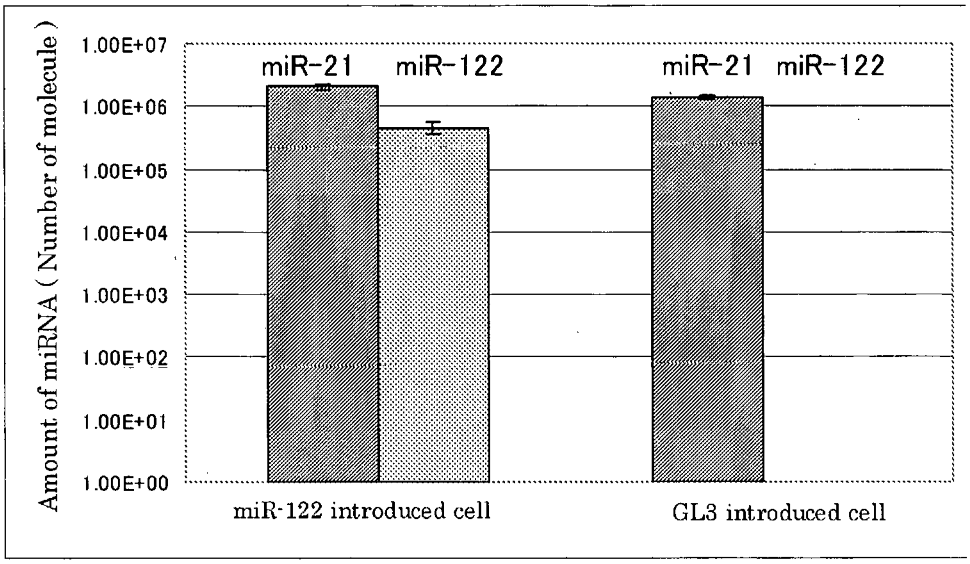
Amount of miRNA ( Number of molecule )
1.00E+07
1.00E+06
1.00E+05
1.00E+04
1.00E+03
1.00E+02
1.00E+01
1.00E+00
miR-21
miR-122
miR-21
miR-122
miR-122 introduced cell
GL3 introduced cell

Fig. 6

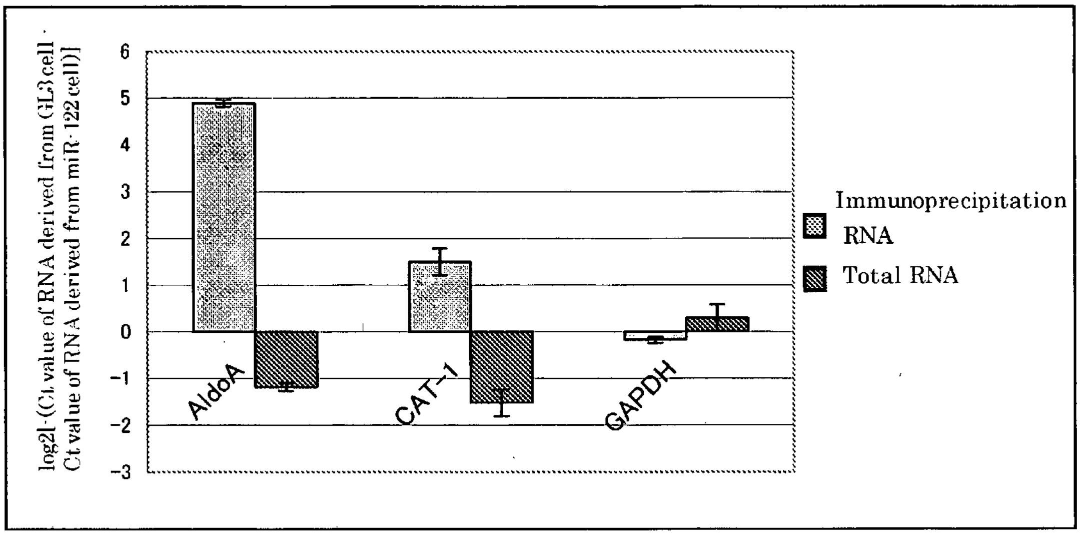
log2[-(Ct value of RNA derived from GL3 cell - Ct value of RNA derived from miR-122 cell)]
6
5
4
3
2
1
0
-1
-2
-3
AldoA
CAT-1
GAPDH
Immunoprecipitation RNA
Total RNA

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007037069 A1
- JP 2006184015 A
- US 5270163 A
- JP 5041946 B
- JP 2007311317 A


### Non-patent literature cited in the description


- **IKEDA et al.** *Journal of Immunological Methods,* 2006, 10417
- **PARK et al.** *Analytical Chemistry,* 2004, vol. 76 (9), 2649-2655
- **SUNAO MATSUHASHI et al.** Introduction of Experimental Immunology. Japan Scientific Society Press, 1981
- **KOHLER ; MILSTEIN.** *Nature,* 1975, vol. 256, 495
- *Eur. J. Immunol.,* 1976, vol. 6, 511
- **JOHNSON.** *Cell,* 2005, vol. 120, 635-647
- **JOACIM.** *Nucleic Acid Research,* 2008, vol. 36 (4
- **SUVENDRA.** *Cell,* 2006, vol. 125, 1111-1124

SEQUENCE LISTING

<110> WAKO PURE CHEMICAL INDUSTRIES, LTD. SUMITOMO BAKELITE CO.,LTD

<120> A carrier for obtaining small RNA and a method of obtaining small RNA and/or target nucleic acid by the carrier

<130> 1743JP

<150> JP2007-311317
<151> 2007-11-30

<160> 11

<170> PatentIn version 3.1

<210> 1
<211> 22
<212> RNA
<213> Artificial

<220>
<223> Olignucleotide

<400> 1
uagcuuauca gacugauguu ga 22

<210> 2
<211> 22
<212> DNA
<213> Artificial

<220>
<223> Olignucleotide primer

<400> 2
aataatagca agtcatttgc gg 22

<210> 3
<211> 20
<212> DNA
<213> Artificial

<220>
<223> Oligonucleotide primer

<400> 3
ccacacatgg caatcccata 20

<210> 4
<211> 23
<212> RNA
<213> Artificial

<220>
<223> Oligonucleotide

<400> 4
uggaguguga caaugguguu ugu 23

<210> 5
<211> 23
<212> RNA
<213> Artificial

<220>
<223> Oligonucleotide

<400> 5
aaacaccauu gucacacucc aua 23

<210> 6
<211> 17
<212> DNA
<213> Artificial

<220>
<223> Oligonucleotide primer

<400> 6
gttgtgggca tcaaggt 17

<210> 7
<211> 19
<212> DNA
<213> Artificial

<220>
<223> Oligonucleotide primer

<400> 7
caatcttcag cacacaacg 19

<210> 8
<211> 17
<212> DNA
<213> Artificial

<220>
<223> Oligonucleotide primer

<400> 8
gttccagagg gagcatc 17

<210> 9
<211> 21
<212> DNA
<213> Artificial

<220>
<223> Oligonucleotide primer

<400> 9
caagcaaata actaccaggt c 21

<210> 10
<211> 19
<212> DNA
<213> Artificial

<220>
<223> Oligonucleotide primer

<400> 10
aatcccatca ccatcttcc 19

<210> 11
<211> 19
<212> DNA
<213> Artificial

<220>
<223> Oligonucleotide primer

<400> 11
gcagagatga tgacccttt 19